(19) Europäisches Patentamt / European Patent Office / Office européen des brevets

(11) **EP 4 483 944 A2**

(12) **EUROPEAN PATENT APPLICATION**

(43) Date of publication:
01.01.2025 Bulletin 2025/01

(21) Application number: 24213702.4

(22) Date of filing: 16.10.2020

(51) International Patent Classification (IPC):
**A61N 1/378** (2006.01)

(52) Cooperative Patent Classification (CPC):
**A61N 1/0556;** A61N 1/36007; A61N 1/36053;
A61N 1/37217; A61N 1/3787

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.10.2019 US 201962916709 P**

(62) Document number(s) of the earlier application(s) in
accordance with Art. 76 EPC:
**20876636.0 / 4 045 133**

(71) Applicant: **Iota Biosciences, Inc.**
**Alameda, CA 94501 (US)**

(72) Inventors:
• **MAHARBIZ, Michel M.**
**Alameda, 94501 (US)**
• **CARMENA, Jose M.**
**Alameda, 94501 (US)**

• **MONTERO GARNIER, Giana**
**Alameda, 94501 (US)**
• **NEELY, Ryan**
**Alameda, 94501 (US)**
• **KAY, Joshua**
**Alameda, 94501 (US)**
• **BOLING, Carl Lance**
**Fremont, 94704 (US)**
• **PASWATERS, Kathryn Mary**
**Fremont, 94704 (US)**

(74) Representative: **V.O.**
**P.O. Box 87930**
**2508 DH Den Haag (NL)**

Remarks:
This application was filed on 18.11.2024 as a
divisional application to the application mentioned
under INID code 62.

(54) **HELICAL NERVE CUFF AND RELATED IMPLANTABLE DEVICES**

(57) A nerve cuff comprises a flexible helical substrate. The flexible helical substrate is configured to at least partially wrap around a filamentous tissue comprising a nerve. The flexible helical substrate is configurable in (a) a flexed position by at least partially unwinding the helical nerve cuff, and (b) a relaxed position. One or more electrodes are positioned along the length of the helical substrate. A handle portion is attached to the helical substrate configured to apply a force that configures the helical substrate in the flexed position.

**EP 4 483 944 A2**

**Description**

CROSS-REFERENCE TO RELATED APPLICATION

This application claims the priority benefit to U.S. Provisional Application No. 62/916,709, filed on October 17, 2019, which is incorporated herein by reference for all purposes.

TECHNICAL FIELD

A helical nerve cuff and an implantable device comprising the helical nerve cuff and a wireless communication system are described herein. Also described are methods of implanting the helical nerve cuff and the implantable device, as well as methods of making the helical nerve cuff and the implantable device.

BACKGROUND

The peripheral nervous system of an individual operates activity of vital organs and physiological homeostasis with tight control. Electrical pulses (i.e., action potentials) transmitted through nerves can alter various physiological functions, such as pulse rates, inflammation, and bladder or bowel control. Certain medical conditions can arise when these neural signals fail to properly control the body, either by over-stimulating or under-stimulating target organs.

Invasive methods have been developed for treating abnormal physiological activity by controlling the electrical signals of the peripheral nervous system. Such methods can include implanting electrodes into the body of a patient, with the tips of the electrodes contacting target nerves. These electrodes generally have long leads that attach to an external device, which subject the patient to substantial risk of infection or displacement of the electrodes. Additionally, because many of the methods are so invasive, certain treatments are limited to clinical settings, and cannot be used as an at-home remedy. Wholly implantable devices have been developed for less invasive treatment, but such devices are too large to be placed in many locations of the body. Therefore, the implanted devices require the use of long leads, which can be displaced or break. Such implanted devices are also implanted to stimulate upstream nerves, such as the vagus nerve, which leads to significant side effects due off target electrical stimulation.

Previously developed nerve cuffs are larger or easily accessible peripheral nerves. However, such nerve cuffs may not be suitable or easily implantable on certain nerves.

SUMMARY OF THE INVENTION

A helical nerve cuff and an implantable device comprising the helical nerve cuff and a body comprising a wireless communication system are described herein.

The body may be attached on the outer surface of the helical nerve cuff. Also described are methods of implanting the helical nerve cuff and the implantable device, as well as methods of making the helical nerve cuff and the implantable device.

An implantable device may comprise a body comprising a wireless communication system; two or more electrodes in electrical communication with the wireless communication system configured to detect an electrophysiological signal transmitted by a nerve or emit an electrical pulse to the nerve; and a helical nerve cuff comprising at least one of the two or more electrodes, wherein the body is on the helical nerve cuff, and the helical nerve cuff is configured to at least partially wrap around a filamentous tissue comprising the nerve and position the at least one of the two or more electrodes in electrical communication with the nerve.

In some embodiments, the wireless communication system comprises an ultrasonic transducer. In some embodiments, the ultrasonic transducer is about 5 mm or less in length in the longest dimension. In some embodiments, the wireless communication system comprises two or more ultrasonic transducers.

In some embodiments, the wireless communication system comprises a radiofrequency antenna.

In some embodiments, the wireless communication system is configured to receive ultrasonic waves or radiofrequency waves, and convert energy from the ultrasonic waves or radiofrequency waves into an electrical energy that powers the device.

In some embodiments, the helical nerve cuff is configured to wrap around the nerve by at least one revolution. In some embodiments, the helical nerve cuff is configured to wrap around the nerve by about 1.3 to about 1.7 revolutions.

In some embodiments, the helical nerve cuff has a width that defines an inner surface, a first edge, and a second edges of the helical nerve cuff, and wherein at least a portion of the first edge contacts at least a portion of the second edge when the helical nerve cuff is in a relaxed position.

In some embodiments, the helical nerve cuff has a width that defines an inner surface, a first edge, and a second edges of the helical nerve cuff, and wherein the first edge does not contact the second edge when the helical nerve cuff is in a relaxed position.

In some embodiments, at least one of the two or more electrodes is positioned along the length of the helical nerve cuff. In some embodiments, the at least one of the two or more electrodes positioned along the length of the helical nerve cuff is positioned on an inner surface of the helical nerve cuff.

At least one of the electrodes on the helical nerve cuff can comprise one or more serpentine segments.

In some embodiments, the helical nerve cuff is flexible and is configurable in (a) a flexed position by at least partially unwinding the helical nerve cuff, and (b) a relaxed position.

In some embodiments, the implantable device further comprises one or more handle portions attached the helical nerve cuff or the body. In some embodiments, the handle portion comprises a loop. In some embodiments, the handle portion comprises a filament. In some embodiments, the handle portion is attached to the nerve cuff at a position proximal to an end of the helical nerve cuff. In some embodiments, the implantable device further comprises a second handle portion attached to the helical nerve cuff at a position proximal to a second end of the helical nerve cuff. In some embodiments, the first handle portion is attached to the second handle. In some embodiments, the device further comprises an additional handle portion attached to a middle position along the length of the helical nerve cuff.

The implantable device can include a mount configured to receive the body and the helical nerve cuff, thereby attaching the body on the helical nerve cuff. Alternatively, the body is directly attached to the helical nerve cuff, such as on an outer surface of the helical nerve cuff or the body is attached to an end of the helical nerve cuff. In some embodiments, the body is attached to middle portion of the helical nerve cuff. In some embodiments, the body comprises an attachment end attached to the helical nerve cuff and an extension end extending from the attachment end.

In some embodiments, the helical nerve cuff comprises a right-handed helical portion. In some embodiments, the helical nerve cuff comprises a left-handed helical portion. In some embodiments, the helical nerve cuff comprises a right-handed helical portion joined to a left-handed helical portion. In some embodiments, the body attaches to the helical nerve cuff at a position proximal to where the right-handed helix portion is joined to the left-handed helical portion. In some embodiments, the right-handed helical portion is joined to a left-handed helical portion through a linear joining portion.

In some embodiments, the helical nerve cuff comprises one or more electrodes configured to emit an electrical pulse to the nerve. In some embodiments, the helical nerve cuff comprises one or more electrodes configured to detect the electrophysiological signal transmitted by the nerve. In some embodiments, the helical nerve cuff comprises two or more electrodes configured to detect the electrophysiological signal transmitted by the nerve.

In some embodiments, the nerve is a human splenic nerve. In some embodiments, the nerve is a human splanchnic nerve.

In some embodiments, the body comprises a housing. In some embodiments, the housing is configured as one of the two or more electrodes. In some embodiments, the housing comprises an acoustic window. In some embodiments, the housing contains an acoustically conductive material.

In some embodiments, the body comprises an integrated circuit electrically connected to the wireless communication system and the two or more electrodes. In some embodiments, the integrated circuit comprises an energy storage circuit comprising a capacitor.

In some embodiments, the body is about 8 mm or less in length in the longest dimension.

In some embodiments, the wireless communication system is configured to transmit data. In some embodiments, the wireless communication system is configured to emit an ultrasonic backscatter or radiofrequency backscatter that encodes the data. In some embodiments, the data comprises information related to a detected electrophysiological signal, a measured physiological condition, a device status, or an emitted electrical pulse.

In some embodiments, the wireless communication system is configured to receive instructions for operating the implantable device. In some embodiments, the instructions are encoded in ultrasonic waves or radiofrequency waves. In some embodiments, the instructions comprise a trigger signal that operates the implantable device to emit an electrical pulse to the nerve.

In some embodiments, the implantable device further comprises a sensor configured to detect a physiological condition. In some embodiments, the sensor is configured to detect temperature, pH, pressure, strain, or an analyte concentration.

In some embodiments, the fibrous tissue comprises a blood vessel.

Also described herein is a system, comprising any one of the implantable devices described above, and an interrogator comprising a second wireless communication system configured to wirelessly communicate with the wireless communication system of the implantable device. In some embodiments, the second wireless communication system comprises one or more ultrasonic transducers configured to transmit ultrasonic waves to the implantable medical device, wherein the ultrasonic waves power the implantable medical device. In some embodiments, the second wireless communication system comprises one or more radiofrequency antennas configured to transmit radiofrequency waves to the implantable medical device, wherein the radiofrequency waves power the implantable medical device. In some embodiments, the interrogator is configured to be worn externally.

Further described herein is a nerve cuff, comprising a flexible helical substrate configured to at least partially wrap around a filamentous tissue comprising a nerve, and is configurable in (a) a flexed position by at least partially unwinding the helical nerve cuff, and (b) a relaxed position; one or more electrodes positioned along the length of the helical substrate; and a handle portion attached to the helical substrate configured to apply a force that configures the helical substrate in the flexed position.

In some embodiments of the helical nerve cuff, the handle portion comprises a loop. In some embodiments, the handle portion comprises a filament. In some embodiments, a portion of the handle portion is embedded within the substrate. In some embodiments, the handle portion is attached to the nerve cuff at a position proximal to an end of the helical nerve cuff. In some embodiments, the helical nerve cuff comprises a second handle portion attached to the helical nerve cuff at a position proximal to a second end of the helical nerve cuff. In some embodiments, the first handle portion and the second handle portion are separated by a radial angle of about 90° to about 180° around a helical axis. In some embodiments, the first handle portion is attached to the second handle portion. In some embodiments, the helical nerve cuff comprises an additional handle portion attached on a middle position along the length of the helical nerve cuff.

The one or more electrodes of the helical nerve cuff can comprise one or more serpentine segments.

In some embodiments, the helical nerve cuff is configured to wrap around the nerve by at least one revolution. In some embodiments, the helical nerve cuff is configured to wrap around the nerve by about 1.3 to about 1.7 revolutions.

In some embodiments, the substrate of the helical nerve cuff has a width that defines an inner surface, a first edge of the substrate, and a second edge of the substrate, and wherein at least a portion of the first edge contacts at least a portion of the second edge when the nerve cuff is in a relaxed position.

In some embodiments, the substrate of the helical nerve cuff has a width that defines an inner surface, a first edge of the substrate, and a second edge of the substrate, and wherein the first edge does not contact the second edge when the nerve cuff is in a relaxed position.

In some embodiments, at least one of the one or more electrodes of the helical nerve cuff is positioned along the length of the helical nerve cuff. In some embodiments, the at least one of the one or more electrodes positioned along the length of the helical nerve cuff is positioned on an inner surface of the helical nerve cuff.

In some embodiments, the helical nerve cuff is configured to be unwound by at least one revolution.

In some embodiments, the helical nerve cuff comprises a right-handed helical portion. In some embodiments, the helical nerve cuff comprises a left-handed helical portion. In some embodiments, the helical nerve cuff comprises a right-handed helical portion joined to a left-handed helical portion. In some embodiments, the first helical portion is joined to a second portion through a connecting member. In some embodiments, the connecting member is a linear connecting member.

In some embodiments, the nerve cuff comprises one or more electrodes configured to emit an electrical pulse to the nerve. In some embodiments, the nerve cuff comprises one or more electrodes configured to detect the electrophysiological signal transmitted by the nerve. In some embodiments, the nerve cuff comprises two or more electrodes configured to detect the electrophysiological signal transmitted by the nerve. In some embodiments, the nerve cuff comprises one or more electrodes configured to detect the electrophysiological signal transmitted by the nerve, and one or more electrodes configured to emit an electrical pulse to the nerve, wherein at least one of the one or more electrodes configured to emit an electrical pulse to the nerve is wider than at least one of the one or more electrodes configured to detect the electrophysiological signal transmitted by the nerve.

In some embodiments of the helical nerve cuff, the helical nerve cuff is configured to at least partially wrap a filamentous tissue comprising a splenic nerve. In some embodiments of the helical nerve cuff, the helical nerve cuff is configured to at least partially wrap a filamentous tissue comprising a splanchnic nerve.

Also described herein is a method of implanting a helical nerve cuff comprising one or more electrodes, comprising: at least partially unwinding the helical nerve cuff; passing an end of the helical nerve cuff behind a fibrous tissue comprising a nerve; and wrapping the helical nerve cuff around the fibrous tissue. In some embodiments, the helical nerve cuff is any one of the helical nerve cuffs described above.

Further described herein is a method of implanting an implantable device comprising a body comprising a wireless communication system on a helical nerve cuff comprising one or more electrodes, the method comprising at least partially unwinding the helical nerve cuff; passing an end of the helical nerve cuff behind a fibrous tissue comprising a nerve; and wrapping the helical nerve cuff around the fibrous tissue. In some embodiments, the method further comprises limiting movement of the body as the end of the helical nerve cuff is passed behind the fibrous tissue. In some embodiments, the body is held in an approximately steady position by holding a handle portion attached to the helical nerve cuff at a position proximal to the body or a handle portion attached to the body. In some embodiments, the implantable device is any one of the implantable devices described above.

In some embodiments of implanting the helical nerve cuff or the implantable device, the method comprises pulling the end of the helical nerve cuff passed behind the fibrous tissue.

In some embodiments of implanting the helical nerve cuff or the implantable device, the method comprises orienting the helical nerve cuff substantially parallel to the fibrous tissue.

In some embodiments of implanting the helical nerve cuff or the implantable device, the helical nerve cuff is at least partially unwound by pulling the end of the helical nerve cuff passed behind the fibrous tissue. In some embodiments of implanting the helical nerve cuff or the implantable device, the helical nerve cuff is at least partially unwound prior to pulling the end of the helical nerve cuff passed behind the fibrous tissue.

In some embodiments of implanting the helical nerve cuff or the implantable device, the end of the helical nerve cuff is passed behind the fibrous tissue from underneath the fibrous tissue.

In some embodiments of implanting the helical nerve cuff or the implantable device, the method comprises separating the fibrous tissue from surrounding tissue. In some embodiments, the fibrous tissue is separated from the surrounding tissue by circumferentially dissecting a portion of the fibrous tissue.

In some embodiments of implanting the helical nerve cuff or the implantable device, the end of the helical nerve cuff passed behind the fibrous tissue is pulled by pulling a handle portion attached to the end of the helical nerve cuff.

In some embodiments of implanting the helical nerve cuff or the implantable device, the fibrous tissue comprises a splenic nerve. In some embodiments of implanting the helical nerve cuff or the implantable device, the fibrous tissue comprises a splanchnic nerve.

Further described herein is a method of making an implantable device, comprising attaching a feedthrough to a housing; positioning a board assembly comprising a wireless communication system in the housing; attaching the housing to a first nerve cuff layer; attaching the first nerve cuff layer to a second nerve cuff layer comprising one or more electrodes; and electrically connecting the one or more electrodes of the nerve cuff to the board assembly through the feedthrough. In some embodiments, the feedthrough is attached to the housing prior to board assembly being positioned into the housing. In some embodiments, the feedthrough is attached to the board assembly before the board assembly is positioned into the housing. In some embodiments, the method comprises sealing the housing. In some embodiments, the method comprises attaching an acoustic window to the housing. In some embodiments, the acoustic window is attached to an open top of the housing after the board assembly is positioned within the housing. In some embodiments, the method comprises assembling the acoustic window by attaching a foil to a frame. In some embodiments, the method comprises filling the housing with an acoustically conductive material. In some embodiments, acoustically conductive material is filled into the housing through a port on the housing, the method comprising sealing the port. In some embodiments, the method comprises attaching the wireless communication system to the board assembly. In some embodiments, the wireless communication system comprises one or more ultrasonic transducers. In some embodiments, the board assembly comprises an integrated circuit electrically connected to the wireless communication system. In some embodiments, the housing is directly attached to the first nerve cuff layer. In some embodiments, an adhesive or a fastener is used to attach the housing to the first nerve cuff layer. In some embodiments, the first nerve cuff layer is helical. In some embodiments, the method comprises attaching one or more handle portions to the body, the first nerve cuff layer, the second nerve cuff layer, or between the first nerve cuff layer and the second nerve cuff layer. In some embodiments, the method comprises making the second nerve cuff layer by embedding one or more electrodes in a substrate material. In some embodiments, the method comprises wrapping the second nerve cuff layer around a mandrel, and attaching the second nerve cuff layer to the first nerve cuff layer while the second nerve cuff layer is wrapped around the mandrel.

BRIEF DESCRIPTION OF THE DRAWINGS

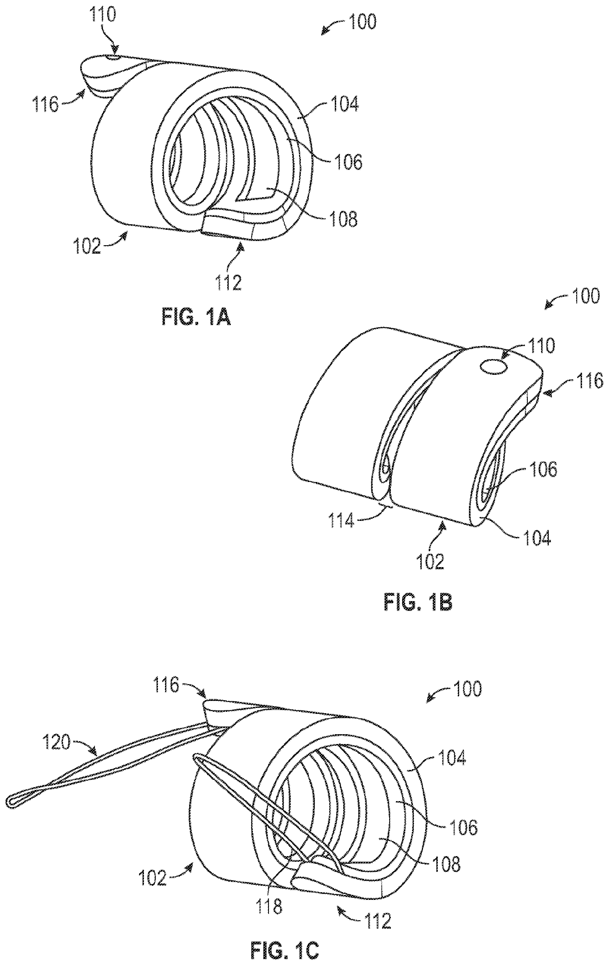

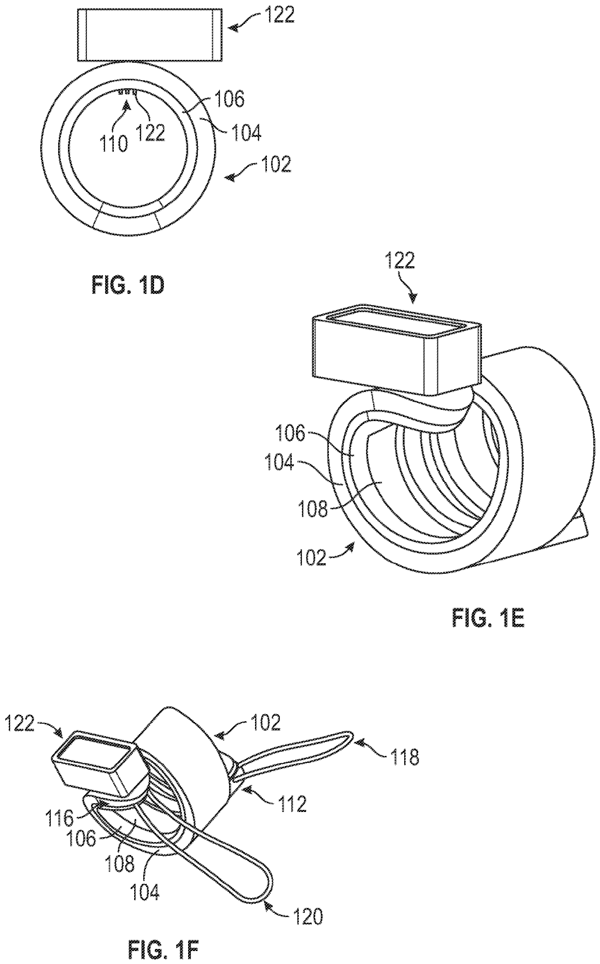

FIG. 1A shows an exemplary helical nerve cuff, which may optionally be part of the implantable device described herein. FIG. 1B shows the nerve cuff illustrated in FIG. 1A from a different angle. FIG. 1C shows an exemplary helical nerve cuff similar to the nerve cuff shown in FIG. 1A and FIG. 1B, but further includes a first handle portion attached to the helical substrate proximal to a first end of the substrate, and a second handle portion attached to the helical substrate proximal to a second end of the substrate. FIG. 1D and FIG. 1E show the helical nerve cuff of FIG. 1A and FIG. 1B attached to a body having a housing. FIG. 1F shows the helical nerve cuff of FIG. 1C attached to a body having a housing.

FIG. 2A and FIG. 2B show front and back perspectives, respectively, of another embodiment of a helical nerve cuff. FIG. 2C shows the helical nerve cuff of FIG. 2A and FIG. 2B attached to a body having a housing.

FIG. 3A and FIG. 3B show front and bottom perspectives, respectively, of another embodiment of a helical nerve cuff. FIG. 3C shows the helical nerve cuff of FIG. 3A and FIG. 3B attached to a body having a housing.

FIG. 4A and FIG. 4B show bottom and top perspectives, respectively, of another embodiment of a helical nerve cuff.

FIG. 5A and FIG. 5B show bottom and top perspectives, respectively, of another embodiment of a helical nerve cuff.

FIG. 6A shows an exemplary helical nerve cuff in a flexed configuration, wherein the helical portions are partially unwound. FIG. 6B shows the helical nerve cuff in FIG. 6B in a relaxed configuration, with the helical portions wound after recoiling from the flexed configuration.

FIG. 7 shows an exemplary board assembly for an implantable device body, which may be enclosed in a housing and attached to a nerve cuff.

FIG. 8 shows a board assembly for a body of a device that includes two orthogonally positioned ultrasonic transducers.

FIG. 9 shows an exemplary body housing attached to a nerve cuff using fasteners.

FIG. 10 shows an exemplary housing with an acoustic window that may be attached to the top of the housing, and a port that may be used to fill the housing with an acoustically conductive material.

FIG. 11A shows an exemplary housing with a feedthrough port at the base of the housing. FIG. 11B shows a housing with a feedthrough attached to the housing. The feedthroughs fit through the feedthrough port, and are brazed, soldered, or otherwise attached to the housing to form a hermetic seal. FIG. 11C shows a cross-sectional view of a device with a housing attached to a nerve cuff. Feedthroughs attached to the housing electrically connect electrodes on the nerve cuff to the board assembly contained within the housing.

FIG. 12 shows an exemplary interrogator that can be used with the implantable device.

FIG. 13 shows an interrogator in communication with an implantable device. The interrogator can transmit ultrasonic waves, which can encode a trigger signal. The implantable device emits an ultrasonic backscatter, which can be modulated by the implantable device to encode information.

FIG. 14 shows a flowchart of an exemplary method for implanting a helical nerve cuff.

FIG. 15 shows a flowchart of another exemplary method for implanting a helical nerve cuff.

FIG. 16 shows a flowchart of an exemplary method for implanting an implantable device having a body on a helical nerve cuff.

FIG. 17 shows a flowchart of another exemplary method for an implantable device having a body on a helical nerve cuff.

FIG. 18 shows a flowchart of an exemplary method of making an implantable device having a body on a helical nerve cuff.

FIG. 19A shows an exemplary implantable device that includes a body attached to the substrate of a helical nerve cuff through a mount. FIG. 19B shows an exemplary helical nerve cuff that can engage a substrate receiving port of a mount. FIG. 19C shows an exemplary mount that includes a substrate receiving port to receive the terminus of a substrate of a helical nerve cuff and a body receiving port to receive a body. When the body and the substrate are both attached to the mount, the body is in electrical communication with the one or more electrodes of the helical nerve cuff.

DETAILED DESCRIPTION

Helical nerve cuffs containing one or more electrodes for stimulating a nerve and/or detecting an electrophysiological signal (such as an action potential, or absence of an action potential) transmitted by a nerve are described herein. Also described are implantable devices that include the helical nerve cuff, which include a body configured to operate the helical nerve cuff. The body includes a wireless communication system, which can wirelessly receive instructions from another device or wirelessly transmit information (such as information about a detected electrophysiological signal or other physiological feature) to another device. Also described are methods of implanting the helical nerve cuff and implantable device, as well as methods of making the helical nerve cuff and implantable device.

The nerve cuff can include a helical substrate configured to at least partially wrap around a filamentous tissue comprising a nerve, and one or more electrodes positioned on an inner surface (for example, along the length) of the helical substrate. The helical nerve cuff can be implanted in a subject such that the electrode is in electrical communication with the nerve. The electrode(s) may be configured to detect an electrophysiological signal transmitted by a nerve or emit an electrical pulse to the nerve. In some embodiments, the nerve cuff includes a handle portion attached to the helical substrate, which may be used to facilitate implantation of the nerve cuff. The helical nerve cuff may be implanted on a small and/or difficult to access nerve of the subject, such as the splenic nerve or a splanchnic nerve, and the helical design of the nerve cuff allows for easier implantation.

The implantable device includes a body having a wireless communication system (which may include, for example, one or more ultrasonic transducers or one or more radiofrequency (RF) antennas), two or more electrodes in electrical communication with the wireless communication system, and the helical nerve cuff. The two or more electrodes are configured to detect an electrophysiological signal transmitted by a nerve or emit an electrical pulse to the nerve, and one or more of the electrodes are positioned on the helical nerve cuff. The body may include an electrically conductive housing, which itself is optionally one of the electrodes.

The helical nerve cuff may be implanted in a subject by circumferentially dissecting a portion of a fibrous tissue comprising a nerve (which may further include a blood vessel, i.e., a neurovascular bundle), threading an end of the nerve cuff through the back side of the fibrous tissue, and rotating the helical nerve cuff to position the nerve cuff around the fibrous tissue. The helical nerve cuff may be attached to a body, and the body of the device may be positioned in front of the fibrous tissue when the cuff is positioned around the fibrous tissue. If the helical nerve cuff includes a handle portion attached to an end of the helical nerve, the handle portion may be threaded through the back side of the fibrous tissue to lead the end of the helical nerve cuff through the back side of the fibrous tissue.

The handle portion attached to the helical nerve cuff allows for carful manipulation of the nerve cuff in the small compartment space of the nerve when nerve cuff is being implanted. A surgical grasping tool can be used to grasp the handle portion to thread it through to the back side of the circumferentially dissected fibrous tissue. In some embodiments, the nerve huff includes a second handle portion on the opposite end of the helical nerve cuff. The second handle portion may also be grasped by a surgical grasping tool to guide the nerve cuff in place, unwind the helix during implantation, or to rotate the helical nerve cuff to correctly position the device.

## Definitions

As used herein, the singular forms "a," "an," and "the" include the plural reference unless the context clearly dictates otherwise.

Reference to "about" or "approximately" a value or parameter herein includes (and describes) variations that are directed to that value or parameter per se. For example, description referring to "about X" includes description of "X."

It is understood that aspects and variations of the invention described herein include "consisting" and/or "consisting essentially of" aspects and variations.

The term "subject" and "patient" are used interchangeably herein to refer to a vertebrate animal.

The terms "treat," "treating," and "treatment" are used synonymously herein to refer to any action providing a benefit to a subject afflicted with a disease state or condition, including improvement in the condition through lessening, inhibition, suppression, or elimination of at least one symptom, delay in progression of the disease or condition, delay in recurrence of the disease or condition, or inhibition of the disease or condition.

Where a range of values is provided, it is to be understood that each intervening value between the upper and lower limit of that range, and any other stated or intervening value in that stated range, is encompassed within the scope of the present disclosure. Where the stated range includes upper or lower limits, ranges excluding either of those included limits are also included in the present disclosure.

It is to be understood that one, some or all of the properties of the various embodiments described herein may be combined to form other embodiments of the present invention. The section headings used herein are for organizational purposes only and are not to be construed as limiting the subject matter described.

Features and preferences described above in relation to "embodiments" are distinct preferences and are not limited only to that particular embodiment; they may be freely combined with features from other embodiments, where technically feasible, and may form preferred combinations of features. The description is presented to enable one of ordinary skill in the art to make and use the invention and is provided in the context of a patent application and its requirements. Various modifications to the described embodiments will be readily apparent to those persons skilled in the art and the generic principles herein may be applied to other embodiments. Thus, the present invention is not intended to be limited to the embodiment shown but is to be accorded the widest scope consistent with the principles and features described herein.

## Helical Nerve Cuff

The helical nerve cuff includes a helical substrate configured to at least partially wrap around a filamentous tissue comprising a nerve, and one or more electrodes positioned along the length of the helical substrate. The nerve cuff may optionally include one or more handle portions, for example a handle portion attached to an end of the helical substrate.

The nerve cuff is configured to at least partially wrap around a filamentous tissue that includes a nerve. The filamentous tissue may include filamentous tissue in addition to the nerve, such as a vessel (e.g., a neurovascular bundle). For example, the splenic nerve is closely associated with the splenic artery and the nerve cuff may be configured to at least partially wrap around both the splenic nerve and the splenic artery. The inner diameter of the helical nerve cuff may be selected based on the diameter of the filamentous tissue, which may different depending on the species of the subject, the target nerve, or other anatomical differences within the subject (e.g., the size of the nerve within the specific subject. By way of example, the inner diameter may be between about 1 mm and about 8 mm in diameter (such as between about 1 mm and about 2 mm, about 2 mm and about 3 mm, about 3 mm and about 4 mm, about 4 mm and about 5 mm, about 5 mm and about 6 mm, about 6 mm and about 7 mm, or about 7 mm and about 8 mm in diameter).

The helical nerve cuff may be configured to wrap around the nerve by at least one revolution. For example, the helical nerve cuff may wrap around the nerve by about 1 to about 4 revolutions, such as about 1 to about 1.3 revolutions, about 1.3 to about 1.7 revolutions, about 1.7 to about 2 revolutions, about 2 to about 2.5 revolutions, about 2.5 to about 3 revolutions, or about 3 to about 4 revolutions. In some embodiments, the helical nerve cuff is configured to wrap around the nerve by about 1.5 revolutions.

The substrate of the nerve cuff is an elongated material wound into a helical shape. The helical substrate may have a substantially flat inner surface and/or a substantially flat outer surface. The width of the substrate may be substantially uniform, with optionally tapered or rounded ends. The width of the substrates define edges, and the edges may or may not contact each other as the substrate winds in the helical shape when the nerve cuff is in a relaxed position. For example, in some embodiments, a gap may or may not separate the revolutions of the substrate. In some embodiments, the substrate has a width that defines an inner surface, a first edge of the substrate, and a second edge of the substrate, and wherein at least a portion of the first edge contacts at least a portion of the second edge when the nerve cuff is in a relaxed position. In some embodiments, the substrate has a width that defines an inner surface, a first edge of the substrate, and a second edge of the substrate, and wherein the first edge does not contact the second edge when the nerve cuff is in a relaxed position.

The substrate of the helical nerve cuff is made from an insulating material, which may be a biocompatible and/or elastomeric material. Exemplary substrate materials include, but are not limited to, silicone, silicone rubber, polydimethylsioloxane (PDMS), a urethane polymer, a poly(p-xylylene)polymer (such as a poly(p-xylylene) polymer sold under the tradename PARYLENE®), or a polyimide.

In some embodiments, the substrate of the helical nerve cuff may include two or more layers, which may be of the same material or of different materials. The layers can included an inner layer that forms the inner surface of the helical nerve cuff and contacts the fibrous tissue, and an outer layer that forms that forms the outer surface of the helical nerve cuff. An electrically conductive material may be positioned between the inner and outer layers, which can form the electrodes of the helical nerve cuff. For example, the inner layer can include one or more opening on the inner surface to expose the electrically conductive material, which defines the electrodes. The separate inner and outer layers can further define the helical shape of the substrate. For example, the inner layer may be under higher tensile force than the outer layer when the helical nerve cuff is in a flexed configuration, which forces the substrate to curl inwards when the helical nerve cuff is in a relaxed configuration.

The width of the nerve cuff can depend on the on the length of nerve cuff (i.e., the maximal distance along an axis running through the center of the helix between the ends of the nerve cuff), the number of revolutions of the substrate, and size of a gap between substrate revolutions (if any). In some embodiments, the length of the nerve cuff is about 4 mm to about 20 mm (such as about 4 mm to about 7 mm, about 7 m to about 10 mm, about 10 mm to about 13 mm, about 13 mm to about 16 mm, or about 16 mm to about 20 mm). In some embodiments, the width (or the inner width) of the substrate is about 2 mm to about 8 mm (such as about 2 mm to about 4 mm, about 4 mm to about 6 mm, or about 6 mm to about 8 mm).

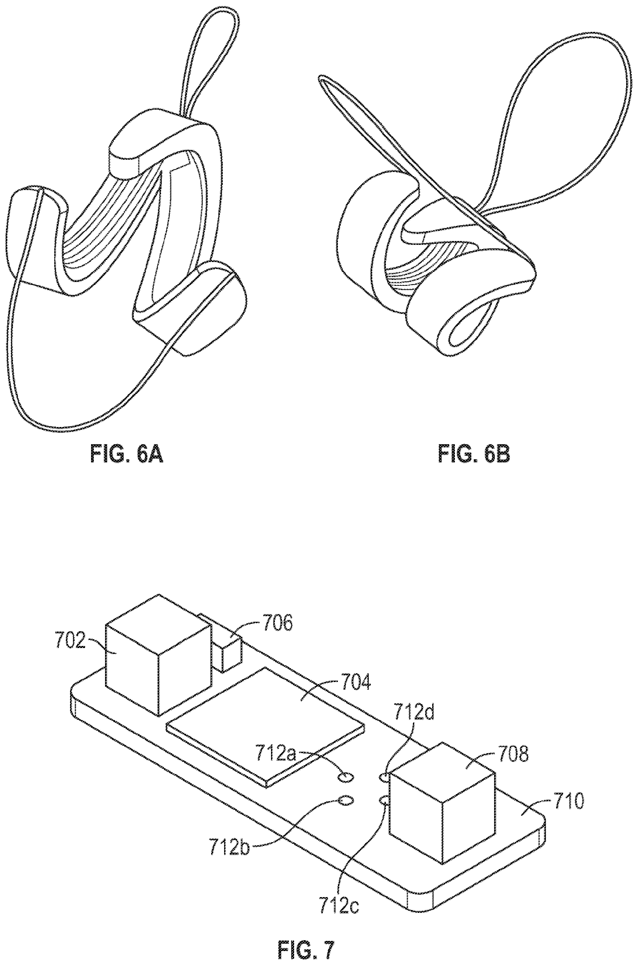

The nerve cuff may be flexible, which allows for manipulation of the nerve cuff upon implantation. For example, in some embodiments, the helical nerve cuff can be configured in a flexed position by at least partially unwinding the helical nerve cuff, and a relaxed position with the helical nerve cuff in a helical configuration. FIG. 6A shows an exemplary helical nerve cuff in an flexed position, wherein both the right-handed helical portion and the left-handed helical portion of the nerve cuff are partially unwound by pulling a first handle portion and a second handle portion which are joined together and attached to either end of the right-handed helical portion and the left-handed helical portion in one direction, and pulling a third handle portion attached to a joining member in the opposite direction. FIG. 6B shows the same helical nerve cuff shown in FIG. 6A in a relaxed position.

The nerve cuff may include a right-handed helical portion, a left-handed helical potion, or both a right-handed helical portion a left-handed helical portion. For example, in some embodiments, the nerve cuff may include a right-handed helical portion joined to a left-handed helical portion, either directly or through a connecting member (which may be linear, curved, or hinged).

The one or more electrodes of the nerve cuff may be positioned on the inner surface of the nerve cuff substrate, and may be uncoated or coated with an electrically conductive material (e.g., electroplated with a poly(3,4-ethylenedioxythiophene) (PEDOT) polymer or other electrically conductive polymer or a metal to improve electrical characteristics of the electrode). In some embodiments, one or more of the electrodes are point electrodes. In some embodiments, one or more of the electrodes may be elongated, and may be positioned, for example, along the length of the helical substrate. The electrodes may terminate before the end of the substrate, at the end of the substrate, or beyond the end of the substrate. The one or more electrodes may be connected to a feed through on the helical nerve cuff, which allows the electrodes to be electrically connected to the outer surface of the substrate or a body attached to the outer surface of the nerve cuff.

The nerve cuff includes one or more electrodes, such as 1, 2, 3, 4, 5, 6, 7, 8, 9, 10 or more electrodes. In some embodiments, one or more of the electrodes are configured to emit an electrical pulse to the nerve. In some embodiments, the nerve cuff includes 1, 2, 3, 4, 5, 6, 7, 8, 9, 10 or more electrodes configured to emit an electrical pulse to the nerve. In some embodiments, one or more of the electrodes are configured to detect an electrophysiological signal transmitted by the nerve. In some embodiments, the nerve cuff includes 1, 2, 3, 4, 5, 6, 7, 8, 9, 10 or more electrodes configured to detect an electrophysiological signal transmitted by the nerve. In some embodiments, one or more electrodes are configured to emit an electrical pulse to the nerve, and one or more of the electrodes are configured to detect an electrophysiological signal transmitted by the nerve. In some embodiments, an electrode configured to emit an electrical pulse is wider than an electrode configured to detect an electrophysiological signal. Electrodes of the helical nerve cuff (having more than one electrode) may be positioned along the length of the helical nerve cuff alongside each other, or in different directions.

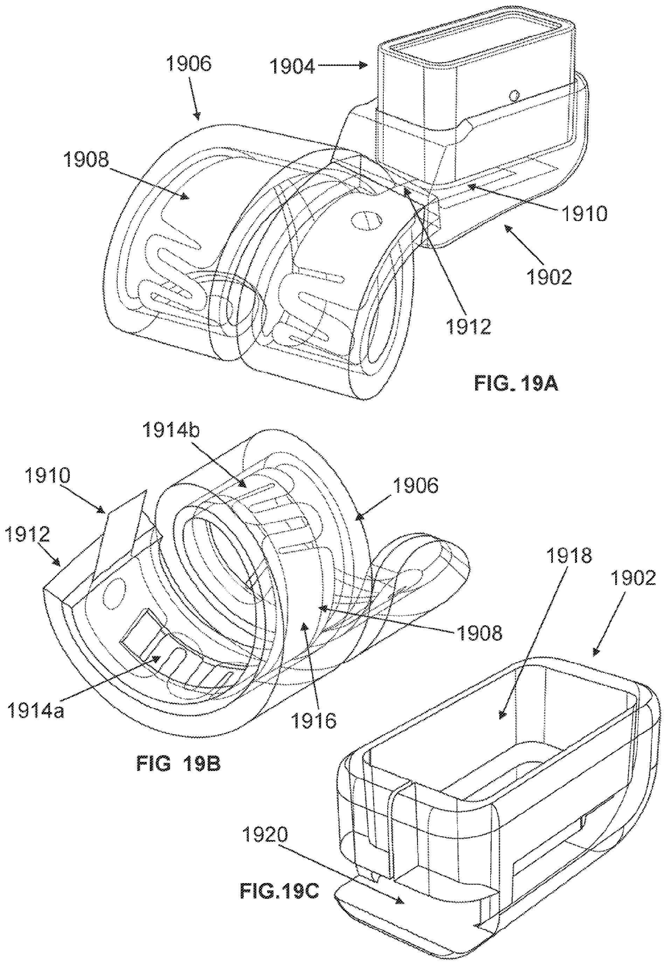

Optionally, one or more of the electrodes on the nerve cuff has a serpentine configuration, or one or more (e.g., 2, 3, 4, or more) serpentine segments. FIG. 19B shows an example of an electrode 1908 with a first serpentine segment 1914a and a second serpentine segment 1914b, separated by a non-serpentine segment 1916. The serpentine configuration of the electrodes allows for enhanced flexibility of the electrode and nerve cuff, which is particularly beneficial when implanting the nerve cuff. Because certain peripheral nerves, such as the splenic nerve, may require substantial maneuvering and flexing of the nerve cuff during implantation and placement, the serpentine configuration can lessen the likelihood of nerve cuff or electrode damage during implantation.

The optional handle portion is configured to be grasped by a surgical grasping tool (e.g., forceps, hook, or other grasping or gripping instrument), and may be useful for manipulating the helical nerve cuff during implantation. The handle portion may extend from or be partially embedded within the helical substrate, and may be more flexible and/or thinner than the substrate to facilitate grasping of the handle portion and manipulation of the nerve cuff. The handle potion may include a loop, for example within the handle portion or by forming a loop by either end of the handle portion being attached to the substrate. In some embodiments, the handle portion comprises a flexible filament (such as a thread, string, cord, suture, or wire), which is optionally biodegradable once implanted within the subject. In some embodiments, the handle portion comprises a bioabsorbable material, such as polyglycolide, polydioxanone, polycaprolactone, or copolymers thereof.

The optional handle portion may be attached to the nerve cuff proximal to the end of the nerve cuff (e.g., at the tip of the helical nerve cuff). The nerve cuff optionally includes more than one handle portion. For example, the helical portion may include an additional handle portion proximal to the opposite end of the helical substrate and/or an additional handle portion proximal to a middle portion of the helical substrate. If the helical nerve cuff is attached to a body, as further discussed herein, the one of the handle portions may be proximal to the body or distal to the body. By way of example, in some embodiments, the body is attached proximal to a first end of the helical nerve cuff, and the handle is attached proximal to the second end of the helical nerve cuff. In some embodiments, the body is attached proximal to a first end of the helical nerve cuff, and the handle portion is attached proximal to the first end of the helical nerve cuff. In some embodiments, a body is attached proximal to a first end of the helical nerve cuff, and a first handle portion is attached proximal to the body and a second handle portion is attached proximal to a second end of the helical nerve cuff. In some embodiments, the body is attached to a middle portion of the helical nerve cuff, a first handle portion is attached proximal to a first end of the helical nerve cuff, a second handle portion is attached proximal to the body, and optionally a third handle portion is attached proximal to the second end of the helical nerve cuff.

Optionally, two or more handle portions attached to the nerve cuff are joined together. For example, a first handle portion includes a first end attached proximal to a first end of the helical nerve cuff, a second handle portion includes a first end attached proximal to a second end of the helical nerve cuff, and the second end of the first handle portion and the second end of the second handle portion are joined together. The first handle portion and the second handle portion may be attached to the helical nerve cuff separated by a radial angle of about 90° to about 180° (for example, about 90° to about 120°, about 120° to about 150°, or about 150° to about 180°) around a helical axis.

FIG. 1A illustrates an exemplary helical nerve cuff, which may optionally be part of the implantable device described herein. FIG. 1B shows the nerve cuff illustrated in FIG. 1A from a different angle. The nerve cuff 100 includes a helical substrate 102 that includes an outer layer 104 and an inner layer 106. The nerve cuff is configured to wrap around the nerve by about 1.5 revolutions, and a gap 114 separates substrate revolutions. The substrate 102 is configured as a left-handed helix, although an embodiment with a right-handed helical substrate is also contemplated. An elongate electrode 108 is positioned on the inner surface of the helical substrate 102. The elongated electrode 108 spans from a feedthrough port 110, and terminates at a position before the end 112 of the helical substrate 102. The electrode 108 is between the outer layer 104 and the inner layer 106, and the inner layer 106 includes an elongated cutout that exposes the electrode 108 to the inner surface of the nerve cuff 100. In an alternative embodiment, the electrode is positioned on top of the inner layer 106. FIG. 1D and FIG. 1E show the helical nerve cuff of FIG. 1A and FIG. 1B attached to a body having a housing 122. The housing 122 is attached to the outer surface of the helical nerve cuff substrate 102. A feedthrough 124 passes through the feedthrough port 110 at electrically connects the elongated electrode 108 to the body.

FIG. 1C illustrates an exemplary helical nerve cuff similar to the nerve cuff illustrated in FIG. 1A and FIG. 1B, but further includes a first handle portion 118 attached to the helical substrate 102 proximal to a first end 112 of the substrate 102, and a second handle portion 120 attached to the helical substrate 102 proximal to a second end 116 of the substrate 102. The first handle portion 118 and the second handle portion 120 are each flexible filaments that form a loop, with each end of the filament attached to the substrate 102. The ends of the filament are embedded within the substrate 102 between the inner layer 106 and the outer layer 104. FIG. 1F shows the helical nerve cuff of FIG. 1C attached to a body having a housing 122. The housing 122 is attached to the outer surface of the helical nerve cuff substrate 102.

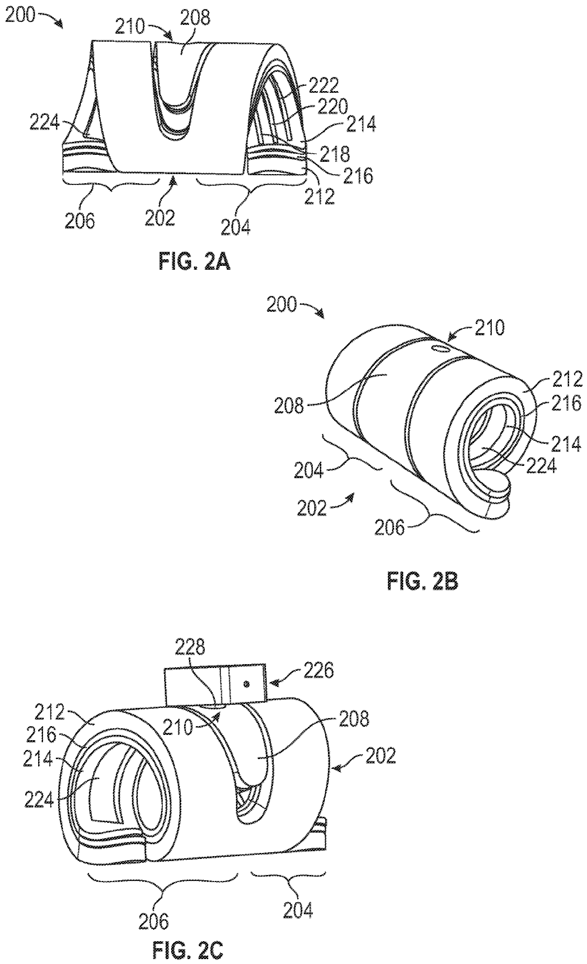

FIG. 2A and FIG. 2B illustrate front and back perspectives, respectively, of another embodiment of a helical nerve cuff 200. The nerve cuff 200 includes a substrate 202 with a left-handed helical segment 204 and a right-handed helical segment 206 joined together through a connecting member 208. The connecting member 208 of the illustrated nerve cuff 200 is a curved and elongated portion of the substrate 202 that makes slightly less than one full rotation around the nerve. A feedthrough port 210 is positioned along the connecting member 208, which allows a body to be electrically connected to electrodes positioned on the inner surface of the substrate. The substrate 202 includes an outer layer 212 and an inner layer 214, which sandwiches and electrically conductive middle layer 216 between the outer layer 212 and the inner layer 214. The helical nerve cuff includes three parallel elongated electrodes (218, 220, and 222) configured to detect an electrophysiological signal transmitted by a nerve on the inner surface of the substrate 202 at the left-handed helical segment 204, and a fourth elongated electrode 224 configured to emit an electrical pulse to the nerve on the inner surface of the substrate 202 at the right-handed helical segment 206. The electrodes are defined by an opening in the inner layer 214. In the illustrated embodiment, the fourth elongated electrode 224 is wider than the electrodes 218, 220, and 222. FIG. 2C shows the helical nerve cuff of FIG. 2A and FIG. 2B attached to a body having a housing 226. The housing 226 is attached to the outer surface of the helical nerve cuff substrate 202 at the connecting member 208. A feedthrough 228 passes through the feedthrough port 210 at electrically connects the electrodes 218, 220, 222, and 224 to the body.

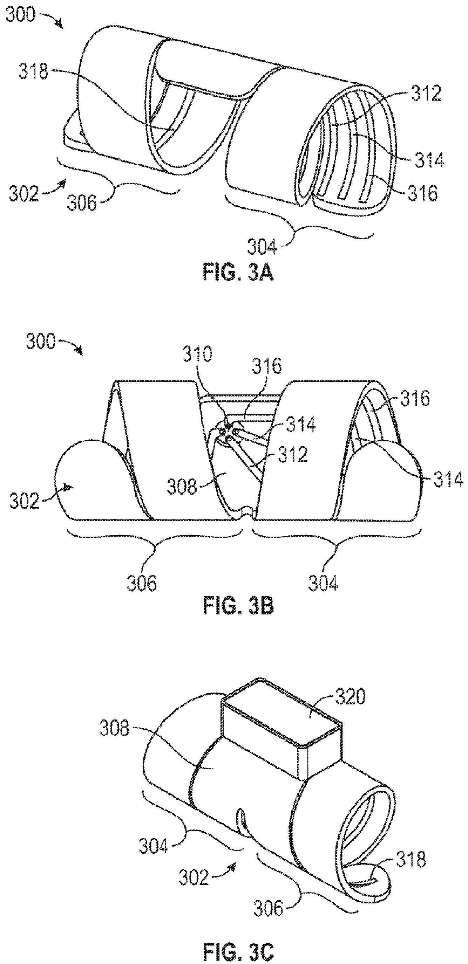

FIG. 3A and FIG. 3B illustrate front and bottom perspectives, respectively, of another embodiment of a helical nerve cuff 300. The nerve cuff 300 includes a substrate 302 with a left-handed helical segment 304 and a right-handed helical segment 306 joined together through a connecting member 308. The connecting member 308 of the illustrated nerve cuff 300 is a curved and elongated portion of the substrate 302, which is shorter than the connecting member of the nerve cuff illustrated in FIG. 2A and FIG. 2B. A feedthrough port 310 is positioned along the connecting member 308, which allows a body to be electrically connected to electrodes positioned on the inner surface of the substrate. The substrate 302 of the illustrated never cuff 300 includes a single layer, with electrodes positioned along the inner surface of the substrate 302. The helical nerve cuff includes three elongated electrodes (312, 314, and 316) on the inner surface of the substrate 302 at the left-handed helical segment 304, and a fourth elongated electrode 318 on the inner surface of the substrate 302 at the right-handed helical segment 306. FIG. 3C shows the helical nerve cuff of FIG. 3A and FIG. 3B attached to a body having a housing 320. The housing 320 is attached to the outer surface of the helical nerve cuff substrate 302.

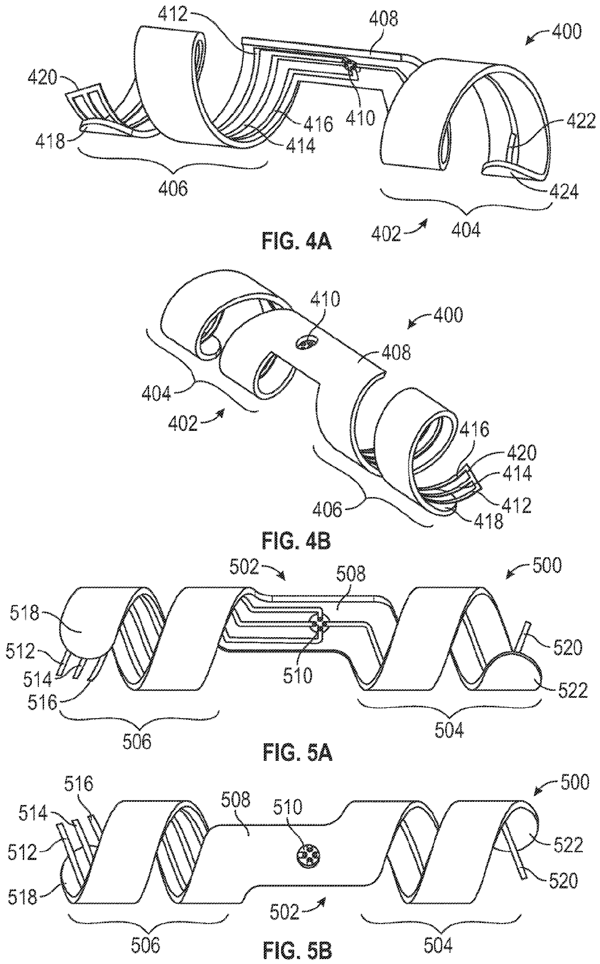

FIG. 4A and FIG. 4B illustrate bottom and top perspectives, respectively, of another embodiment of a helical nerve cuff 400. The nerve cuff 400 includes a substrate 402 with a left-handed helical segment 404 and a right-handed helical segment 406 joined together through a connecting member 408. The connecting member 408 of the illustrated nerve cuff 400 is an elongated and linear connecting member. A feedthrough port 410 is positioned along the connecting member 408, which allows a body to be electrically connected to electrodes positioned on the inner surface of the substrate. The substrate 402 of the illustrated never cuff 400 includes a single layer, with electrodes positioned along the inner surface of the substrate 402. The helical nerve cuff includes three parallel elongated electrodes (412, 414, and 416) on the inner surface of the substrate 402 at the left-handed helical segment 404, and extend beyond the end 418 of the nerve cuff 400. In the illustrated embodiment, the electrodes 412, 414, and 416 which are joined together at a joining end 420. The nerve cuff further includes a fourth elongated electrode 422 on the inner surface of the substrate 402 at the right-handed helical segment 406, which extends beyond the opposite end 424 of the nerve cuff 400.

FIG. 5A and FIG. 5B illustrate bottom and top perspectives, respectively, of another embodiment of a helical nerve cuff 500. The nerve cuff 500 includes a substrate 502 with a first left-handed helical segment 504 and a second left-handed helical segment 506 joined together through a connecting member 508. The connecting member 508 of the illustrated nerve cuff 500 is an elongated and linear connecting member. A feedthrough port 510 is positioned along the connecting member 508, which allows a body to be electrically connected to electrodes positioned on the inner surface of the substrate. The substrate 502 of the illustrated never cuff 500 includes a single layer, with electrodes positioned along the inner surface of the substrate 502. The helical nerve cuff includes three parallel elongated electrodes (512, 514, and 516) on the inner surface of the substrate 502 at the first left-handed helical segment 504, and extend beyond the end 518 of the nerve cuff 500. The nerve cuff further includes a fourth elongated electrode 520 on the inner surface of the substrate 502 at the second left-handed helical segment 506, which extends beyond the opposite end 522 of the nerve cuff 500.

The helical nerve cuff can optionally include a mount configured to receive a body. The mount provide additional stability to the device so that the body does not disconnect from the helical nerve cuff. The mount can attach to the substrate of the helical nerve cuff, for example on the outer surface of the helical nerve cuff or at the end of the helical nerve cuff. The mount can include a substrate receiving port sized and shaped to receive the end of the substrate of the helical nerve cuff. The one or more electrodes of the helical nerve cuff may extend beyond a terminus of the substrate such that they can be positioned deeper within the substrate receiving port than the substrate itself. The mount can also include a body receiving port sized and shaped to receive a body or housing containing the body. The mount can therefore provide stable attachment of the body to the substrate of the helical nerve cuff. As further described herein, the body can include components for operating the one or more electrodes on the helical nerve cuff. Accordingly, the mount provides an electrical connection between the body and the one or more electrodes of the helical nerve cuff. This may occur, for example, by allowing a direct connection between the one or more electrodes of the helical nerve cuff and the body. For example, the mount may include an opening that connects the substrate receiving port and the body receiving port to allow for the electrical connection between the body and the one or more electrodes. Alternatively, the mount can includes one or more electrical feedthroughs that intermediate the electrical connection between the body and the one or more electrodes on the helical nerve cuff.

The mount can be made of an insulating material, which may be a biocompatible and/or elastomeric material. Exemplary materials for the mount include, but are not limited to, silicone, silicone rubber, polyether ether ketone (PEEK), polydimethylsioloxane (PDMS), a urethane polymer, a poly(p-xylylene)polymer (such as a poly(p-xylylene) polymer sold under the tradename PARYLENE®), or a polyimide.

FIGS. 19A-19C show an exemplary helical nerve cuff with a mount 1902 configured to receive a body 1904 attached to a substrate 1906. The substrate 1906 is shown as transparent so that the electrode 1908 on the inner surface of the helical substrate is visible. Referring to FIG. 19C, the mount 1902 includes a substrate receiving port 1920 and a body receiving port 1918. As shown in FIG. 19B, the substrate 1906 can include a terminus 1912, which can be received by the substrate receiving port 1920 of the mount 1902. The electrode 1908 can include an extending portion 1910 that extends beyond the terminus 1912 of the substrate 1906. When the mount is attached to the substrate, the extending portion 1910 of the electrode 1908 is positioned deeper within the substrate receiving port 1920 than the terminus 1912 (see FIG. 19A). Attaching the body 1904 through the body receiving port 1918 allows the electrical connection between the body 1904 and the electrode 1908 through the extending portion 1910 of the electrode 1908.

In some embodiments, the helical nerve cuff is implanted in a subject. The subject can be for example, a mammal. In some embodiments, the subject is a human, dog, cat, horse, cow, pig, sheep, goat, monkey, or a rodent (such as a rat or mouse). The helical nerve cuff may be configured to at least partially wrap around a filamentous tissue (such as a peripheral nerve, or a filamentous tissue comprising a peripheral nerve, such as a neurovascular bundle) within any of these animals, or others. For example, in some embodiments, the helical nerve cuff is configured to at least partially wrap around a human peripheral nerve, such as a human splenic nerve, or a human splenic neurovascular bundle. In some embodiments, the helical nerve cuff is configured to at least partially wrap around a human peripheral nerve, such as a human splanchnic nerve. In some embodiments, the helical nerve cuff is configured to at least partially wrap around an autonomic nerve. In some embodiments, the nerve is a sympathetic nerve. In some embodiments, the nerve is a vagus nerve, a mesenteric nerve, a splenic nerve, a sciatic nerve, a tibial nerve, a pudendal nerve, a celiac ganglion, a sacral nerve, or any branch thereof.

**Implantable Device**

An implantable device, which may be used to detect and electrophysiological signal transmitted by a nerve or emit an electrical pulse to a nerve, can include a body attached to a helical nerve cuff. The body is attached to the helical nerve cuff without an interceding lead between the body and the helical nerve cuff. That is, the body is directly attached to the outer surface of the helical nerve cuff to join the body to the nerve cuff. By securing the body to the helical nerve cuff, the never cuff and the body may be simultaneously positioned upon implantation. The body may include a wireless communication system, which is electrically connected to two or more electrodes. The two or more electrodes can be configured to detect an electrophysiological signal transmitted by a nerve or emit an electrical pulse to the nerve, and at least one of the electrodes is included on the helical nerve cuff. The helical nerve cuff may be, for example, a helical nerve cuff as described in further detail herein. The implantable device is full implantable; that is, no wires or leads connect outside the body of the subject after implantation.

The two or more electrodes of the device, including one or more of the electrodes on the helical nerve cuff, are electrically connected to the wireless communication system. The body of the device may further include an integrated circuit, and the electrodes are connected to the wireless communication system through integrated circuit. The integrated circuit may be configured to operate the wireless communication system of the device body, and can operate the two or more electrodes of the implantable device to detect the electrophysiological signal and/or emit an electrical pulse. Optionally, the implantable device includes one or more sensors configured to detect a physiological condition (such as temperature sensor, an oxygen sensor, a pH sensor, a strain sensor, a pressure sensor, an impedance sensor, or a sensor that can detect a concentration of an analyte).

The body of the implantable device includes a wireless communication system, which can communicate with a separate device (such as an external interrogator or another implantable device). For example, the wireless communication may be configured to receive instructions for emitting one or more electrical pulses to the nerve and/or transmit information, such as data associated with detected electrophysiological signal transmitted by the nerve and/or data associated one or more physiological conditions (e.g., pulse, temperature, pressure, presence or concentration of an analyte, etc.). The wireless communication system can include, for example one or more ultrasonic transducers or one or more radiofrequency antennas. The wireless communication system may also be configured to receive energy (for example, through ultrasonic or radiofrequency (RF)) from another device, which can be used to power the implantable device.

Information about the detected electrophysiological signal or physiological condition may be transmitted using the wireless communication system to a receiving device. For example, the wireless communication system may include two or more ultrasonic transducers, which can be operated to encode information about the detected electrophysiological signal or physiological condition using ultrasonic backscatter waves or radiofrequency backscatter waves. Exemplary implantable devices that can detect an electrophysiological signal and encode information related to the detected electrophysiological signal are described in WO 2018/009910 A2. Exemplary implantable devices that can be operated using ultrasonic waves to emit an electrical pulse are described in WO 2018/009912 A2. Exemplary implantable devices that are powered by ultrasonic waves and can emit an ultrasonic backscatter encoding a detected physiological condition are described in WO 2018/009905 A2 and WO 2018/009911 A2.

An integrated circuit included in the device body can electrically connect and communicate between the electrodes or sensor and the wireless communication system (e.g., the one or more ultrasonic transducers or one or more RF antennas). The integrated circuit can include or operate a modulation circuit within the wireless communication system, which modulates an electrical current flowing through the wireless communication system (e.g., one or more ultrasonic transducers or one or more radiofrequency antennas) to encode information in the electrical current. The modulated electrical current affects backscatter waves (e.g., ultrasonic backscatter waves or radiofrequency backscatter waves) emitted by the wireless communication system, and the backscatter waves encode the information.

FIG. 7 shows a side view of a board assembly of an exemplary implantable device body, which may be surrounded by a housing, and can be attached to a helical nerve cuff. The body includes a wireless communication system (e.g., an ultrasonic transducer) 702 and an integrated circuit 704. In the illustrated embodiment, the integrated circuit 704 includes a power circuit that includes a capacitor 706. In the illustrated embodiment, the capacitor is an "off chip" capacitor (in that it is not on the integrated circuit chip), but is still electrically integrated into the circuit. The capacitor can temporarily store electrical energy converted from energy (e.g., ultrasonic waves) received by the wireless communication system, and can be operated by the integrated circuit 704 to store or release energy. Optionally, the body further includes a sensor 708, configured to detect a physiological condition. The ultrasonic transducer 702, integrated circuit 704, the capacitor 706, and the optional sensor 708 are mounted on a circuit board 710, which may be a printed circuit board. The circuit board 710 may further include one or more feedthroughs 712a, 712b, 712c, and 712d that electrically connect the circuit board and/or integrated circuit to one or more electrodes of the helical nerve cuff. The wireless communication system 702 is electrically connected to the integrated circuit 704, and the integrated circuit 704 is electrically connected to the electrodes via the feedthroughs 712a, 712b, 712c, and 712d, thereby electrically connecting the wireless communication system 702 to the electrodes.

The wireless communication system can be configured to receive instructions for operating the implantable device. The instructions may be transmitted, for example, by a separate device, such as an interrogator. By way of example, ultrasonic waves received by the implantable device (for example, those transmitted by the interrogator) can encode instructions for operating the implantable device. In another example, RF waves received by the implantable device can encode instructions for operating the implantable device. The instructions may include, for example, a trigger signal that instructs the implantable device to emit an electrical pulse through the electrodes of the device. The trigger signal may include, for example, information relating to when the electrical pulse should be emitted, a pulse frequency, a pulse power or voltage, a pulse shape, and/or a pulse duration.

In some embodiments, the implantable device can also be operated to transmit information (i.e., uplink communication), which can be received by the interrogator, through the wireless communication system. In some embodiments, the wireless communication system is configured to actively generate a communication signal (e.g., ultrasonic waves or radiofrequency waves) that encode the information. In some embodiments, the wireless communication system is configured to transmit information encoded on backscatter waves (e.g., ultrasonic backscatter waves or RF backscatter waves). Backscatter communication provides a lower power method of transmitting information, which is particularly beneficial for small devices to minimize energy sues. By way of example, the wireless communication system may include one or more ultrasonic transducers configured to receive ultrasonic waves and emit an ultrasonic backscatter, which can encode information transmitted by the implantable device. Current flows through the ultrasonic transducer, which can be modulated to encode the information. The current may be modulated directly, for example by passing the current through a sensor that modulates the current, or indirectly, for example by modulating the current using a modulation circuit based on a detected physiological condition or an electrophysiological pulse.

In some embodiments, the information transmitted by the wireless communication system includes information unrelated to a detected physiological condition or electrophysiological pules detected by the implantable device. For example, the information can include one or more of: information related to the status of the implantable device or a confirmation signal that confirms an electrical pulse was emitted, the power, frequency, voltage, duration, or other information related to an emitted electrical pulse, and/or an identification code for the implantable device. Optionally, the integrated circuit is configured to digitize the information, and the wireless communication system can transmit the digitized information.

The information wirelessly transmitted using the wireless communication system can be received by an interrogator. In some embodiments, the information is transmitted by being encoded in backscatter waves (e.g., ultrasonic backscatter or radiofrequency backscatter). The backscatter can be received by the interrogator, for example, and deciphered to determine the encoded information. Additional details about backscatter communication are provided herein, and additional examples are provided in WO 2018/009905; WO 2018/009908; WO 2018/009910; WO 2018/009911; WO 2018/009912; International Patent Application No. PCT/US2019/028381; International Patent Application No. PCT/US2019/028385; and International Patent Application No. PCT/2019/048647; each of which is incorporated herein by reference for all purposes. The information can be encoded by the integrated circuit using a modulation circuit. The modulation circuit is part of the wireless communication system, and can be operated by or contained within the integrated circuit.

An interrogator can transmit energy waves (e.g., ultrasonic waves or radiofrequency waves), which are received by the wireless communication system of the device to generate an electrical current flowing through the wireless communication system (e.g., to generate an electrical current flowing through the ultrasonic transducer or the radiofrequency antenna). The flowing current can then generate backscatter waves that are emitted by the wireless communication system. The modulation circuit can be configured to modulate the current flowing through the wireless communication system to encode the information. For example, the modulation circuit may be electrically connected to an ultrasonic transducer, which received ultrasonic waves from an interrogator. The current generated by the received ultrasonic waves can be modulated using the modulation circuit to encode the information, which results in ultrasonic backscatter waves emitted by the ultrasonic transducer to encode the information. A similar approach may be taken with a radiofrequency antenna that receives radiofrequency waves. The modulation circuit includes one or more switches, such as an on/off switch or a field-effect transistor (FET). An exemplary FET that can be used with some embodiments of the implantable device is a metal-oxide-semiconductor field-effect transistor (MOSFET). The modulation circuit can alter the impedance of a current flowing through the wireless communication system, and variation in current flowing through the wireless communication system encodes the information. In some embodiments, information encoded in the backscatter waves includes information related to an electrophysiological signal transmitted by the nerve, an electrical pulse emitted by the implantable device, or a physiological condition detected by a sensor of the implantable device. In some embodiments, information encoded in the backscatter waves includes a unique identifier for the implantable device. This can be useful, for example, to ensure the interrogator is in communication with the correct implantable device when a plurality of implantable devices is implanted in the subject. In some embodiments, the information encoded in the backscatter waves includes a verification signal that verifies an electrical pulse was emitted by the implantable device. In some embodiments, the information encoded in the backscatter waves includes an amount of energy stored or a voltage in the energy storage circuit (or one or more capacitors in the energy storage circuit). In some embodiments, the information encoded in the backscatter waves includes a detected impedance. Changes in the impedance measurement can identify scarring tissue or degradation of the electrodes over time.

In some embodiments, the modulation circuit is operated using a digital circuit or a mixed-signal integrated circuit (which may be part of the integrated circuit), which can actively encode the information in a digitized or analog signal. The digital circuit or mixed-signal integrated circuit may include a memory and one or more circuit blocks, systems, or processors for operating the implantable device. These systems can include, for example, an onboard microcontroller or processor, a finite state machine implementation, or digital circuits capable of executing one or more programs stored on the implant or provided via ultrasonic communication between interrogator and implantable device. In some embodiments, the digital circuit or a mixed-signal integrated circuit includes an analog-to-digital converter (ADC), which can convert analog signal encoded in the ultrasonic waves emitted from the interrogator so that the signal can be processed by the digital circuit or the mixed-signal integrated circuit. The digital circuit or mixed-signal integrated circuit can also operate the power circuit, for example to generate the electrical pulse to stimulate the tissue. In some embodiments, the digital circuit or the mixed-signal integrated circuit receives the trigger signal encoded in the ultrasonic waves transmitted by the interrogator, and operates the power circuit to discharge the electrical pulse in response to the trigger signal.

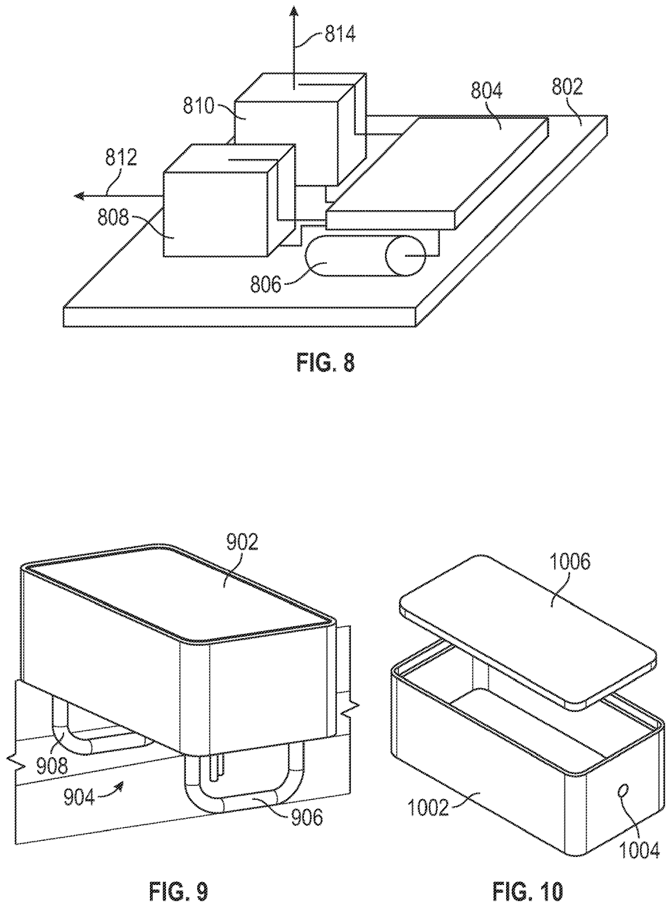

In some embodiments, the wireless communication system includes one or more ultrasonic transducers, such as one, two, or three or more ultrasonic transducers. In some embodiments, the wireless communication system includes a first ultrasonic transducer having a first polarization axis and a second ultrasonic transducer having a second polarization axis, wherein the second ultrasonic transducer is positioned so that the second polarization axis is orthogonal to the first polarization axis, and wherein the first ultrasonic transducer and the second ultrasonic transducer are configured to receive ultrasonic waves that power the device and emit an ultrasonic backscatter. In some embodiments, the wireless communication system includes a first ultrasonic transducer having a first polarization axis, a second ultrasonic transducer having a second polarization axis, and a third ultrasonic transducer having a third polarization axis, wherein the second ultrasonic transducer is positioned so that the second polarization axis is orthogonal to the first polarization axis and the third polarization axis, wherein the third ultrasonic transducer is positioned so that the third polarization axis is orthogonal to the first polarization and the second polarization axis, and wherein the first ultrasonic transducer and the second ultrasonic transducer are configured to receive ultrasonic waves that power the device and emit an ultrasonic backscatter. FIG. 8 shows a board assembly of a body of a device that includes two orthogonally positioned ultrasonic transducers. The body includes a circuit board 802, such as a printed circuit board, and an integrated circuit 804, which a power circuit that includes a capacitor 806. The body further includes a first ultrasonic transducer 808 electrically connected to the integrated circuit 804, and a second ultrasonic transducer 810 electrically connected to the integrated circuit 804. The first ultrasonic transducer 808 includes a first polarization axis 812, and the second ultrasonic transducer 810 includes a second polarization axis 814. The first ultrasonic transducer 808 and the second ultrasonic transducer are positioned such that the first polarization axis 812 is orthogonal to the second polarization axis 814.

The ultrasonic transducer, if included in the wireless communication system, can be a micro-machined ultrasonic transducer, such as a capacitive micro-machined ultrasonic transducer (CMUT) or a piezoelectric micro-machined ultrasonic transducer (PMUT), or can be a bulk piezoelectric transducer. Bulk piezoelectric transducers can be any natural or synthetic material, such as a crystal, ceramic, or polymer. Exemplary bulk piezoelectric transducer materials include barium titanate (BaTiO3), lead zirconate titanate (PZT), zinc oxide (ZO), aluminum nitride (AlN), quartz, berlinite (AlPO4), topaz, langasite (La3Ga5SiO14), gallium orthophosphate (GaPO4), lithium niobate (LiNbO3), lithium tantalite (LiTaO3), potassium niobate (KNbO3), sodium tungstate (Na2WO3), bismuth ferrite (BiFeO3), polyvinylidene (di)fluoride (PVDF), and lead magnesium niobate-lead titanate (PMN-PT).

In some embodiments, the bulk piezoelectric transducer is approximately cubic (i.e., an aspect ratio of about 1:1:1 (length: width: height). In some embodiments, the piezoelectric transducer is plate-like, with an aspect ratio of about 5:5:1 or greater in either the length or width aspect, such as about 7:5:1 or greater, or about 10:10:1 or greater. In some embodiments, the bulk piezoelectric transducer is long and narrow, with an aspect ratio of about 3:1:1 or greater, and where the longest dimension is aligned to the direction of the ultrasonic backscatter waves (i.e., the polarization axis). In some embodiments, one dimension of the bulk piezoelectric transducer is equal to one half of the wavelength ($\lambda$) corresponding to the drive frequency or resonant frequency of the transducer. At the resonant frequency, the ultrasound wave impinging on either the face of the transducer will undergo a 180° phase shift to reach the opposite phase, causing the largest displacement between the two faces. In some embodiments, the height of the piezoelectric transducer is about 10 $\mu$m to about 1000 $\mu$m (such

as about 40 $\mu$m to about 400 $\mu$m, about 100 $\mu$m to about 250 $\mu$m, about 250 $\mu$m to about 500 $\mu$m, or about 500 $\mu$m to about 1000 $\mu$m). In some embodiments, the height of the piezoelectric transducer is about 5 mm or less (such as about 4 mm or less, about 3 mm or less, about 2 mm or less, about 1 mm or less, about 500 $\mu$m or less, about 400 $\mu$m or less, 250 $\mu$m or less, about 100 $\mu$m or less, or about 40 $\mu$m or less). In some embodiments, the height of the piezoelectric transducer is about 20 $\mu$m or more (such as about 40 $\mu$m or more, about 100 $\mu$m or more, about 250 $\mu$m or more, about 400 $\mu$m or more, about 500 $\mu$m or more, about 1 mm or more, about 2 mm or more, about 3 mm or more, or about 4 mm or more) in length. In some embodiments, the ultrasonic transducer has a length of about 5 mm or less such as about 4 mm or less, about 3 mm or less, about 2 mm or less, about 1 mm or less, about 500 $\mu$m or less, about 400 $\mu$m or less, 250 $\mu$m or less, about 100 $\mu$m or less, or about 40 $\mu$m or less) in the longest dimension. In some embodiments, the ultrasonic transducer has a length of about 20 $\mu$m or more (such as about 40 $\mu$m or more, about 100 $\mu$m or more, about 250 $\mu$m or more, about 400 $\mu$m or more, about 500 $\mu$m or more, about 1 mm or more, about 2 mm or more, about 3 mm or more, or about 4 mm or more) in the longest dimension.

The ultrasonic transducer, if included in the wireless communication system, can be connected two electrodes to allow electrical communication with the integrated circuit. The first electrode is attached to a first face of the transducer and the second electrode is attached to a second face of the transducer, wherein the first face and the second face are opposite sides of the transducer along one dimension. In some embodiments, the electrodes comprise silver, gold, platinum, platinum-black, poly(3,4-ethylenedioxythiophene (PEDOT), a conductive polymer (such as conductive PDMS or polyimide), or nickel. In some embodiments, the axis between the electrodes of the transducer is orthogonal to the motion of the transducer.

The implantable device may be configured to wirelessly receive energy and convert the energy into an electrical energy, which may be used to power the device. The wireless communication system may be used to wireless receive the energy, or a separate system may be configured to receive the energy. For example, an ultrasonic transducer (which may be an ultrasonic transducer contained within the wireless communication system or a different ultrasonic transducer) can be configured to receive ultrasonic waves and convert energy from the ultrasonic waves into an electrical energy. In some embodiments, an RF antenna (which may be an RF antenna contained within the wireless communication system or a different RF antenna) is configured to receive RF waves and convert the energy from the RF waves into an electrical energy. The electrical energy is transmitted to the integrated circuit to power the device. The electrical energy may power the device directly, or the integrated circuit may operate a power circuit to store the energy for later use.

In some embodiments, the integrated circuit includes a power circuit, which can include an energy storage circuit. The energy storage circuit may include a battery, or an alternative energy storage device such as one or more capacitors. The implantable device is preferably batteryless, and may instead rely on one or more capacitors. By way of example, energy from ultrasonic waves or radiofrequency waves received by the implantable device (for example, through the wireless communication system) is converted into a current, and can be stored in the energy storage circuit. The energy can be used to operate the implantable device, such as providing power to the digital circuit, the modulation circuit, or one or more amplifiers, or can be used to generate the electrical pulse used to stimulate the tissue. In some embodiments, the power circuit further includes, for example, a rectifier and/or a charge pump.

The integrated circuit may be configured to operate the two or more electrodes of the device configured to detect an electrophysiological signal transmitted by a nerve or emit an electrical pulse to the nerve, and at least one of the electrodes is included on the helical nerve cuff. The electrodes may be positioned on the helical nerve cuff, the body of the device, or both (e.g., one or more electrodes may be on the body of the device and one or more electrodes may be on the helical nerve cuff). In some embodiments, the housing of the body operates as an electrode. For example, the device may include one or more working electrodes on the helical nerve cuff, and the housing may be configured as a counter electrode. Accordingly, in some embodiments, the housing of the device is electrically connected to the integrated circuit. The one or more electrodes on the helical nerve cuff are electrically connected to the integrated circuit, for example through one or more feedthroughs.

In some embodiments, the implantable device includes one or more sensors configured to detect a physiological condition. The sensor(s) may be, for example, included as part of the body of the device or on the helical nerve cuff. The sensors are configured to detect a physiological condition, such as temperature, oxygen concentration, pH, an analyte (such as glucose), strain, or pressure. Variation in the physiological condition modulates impedance, which in turn modulates current flowing through a detection circuit electrically connected to or part of the integrated circuit. The implantable device may comprise one or more (such as 2, 3, 4, 5 or more) sensors, which may detect the same physiological condition or different physiological conditions. In some embodiments, the implantable device comprises 10, 9, 8, 7, 6 or 5 or fewer sensors). For example, in some embodiments, the implantable device comprises a first sensor configured to detect temperature and a second sensor configured to detect oxygen. Changes in both physiological conditions can be encoded in the backscatter waves emitted by the wireless communication system, which can be deciphered by an external computing system (such as the interrogator).

The body of the implantable device is attached to the helical nerve cuff, for example on the outer surface of the helical nerve cuff. In some embodiments, the body is attached to an end of the helical nerve cuff, or at a middle portion of the helical nerve cuff. Optionally, a handle portion may be attached to the helical nerve cuff, and may be attached at a position proximal to the body. In some embodiments, the implantable device includes a handle portion attached to the helical nerve cuff at a position proximal to the body attached to the helical nerve cuff, and a second handle portion attached to the helical nerve cuff at a distal position, such as at an end of the helical nerve cuff. Examples of an implantable device body attached to a helical nerve cuff is shown in FIGS. 1D, 1E, 1F, 2C, and 3C. In some embodiments, a handle portion is attached to the body of the implantable device.

The body (or housing) of the of the implantable device may be attached to the helical nerve cuff through an adhesive (e.g., an epoxy, glue, cement, solder, or other binder), one or more fasteners (e.g., a staple, screw, bolt, clap, rivet, pin, rod, etc.), or any other suitable means to securely attach the body to the helical nerve cuff to ensure that it does not become separated from the nerve cuff after implantation. The body (or housing) may be directly attached to the nerve cuff. FIG. 9 illustrates an exemplary body 902 attached to a nerve cuff 904 using fasteners (906 and 908). In some embodiment, the body has an elongated shape, and one end of the body (i.e., the attachment end) is attached to the helical nerve cuff, and the opposite end (i.e., an extension end) extends from the nerve cuff (see, for example, the body attached to the nerve cuff in FIG. 1E).

The body can include a housing, which can include a base, one or more sidewalls, and a top. The housing is optionally made from an electrically conductive material and may be configured as one of the one or more electrodes of the implantable device configured to detect an electrophysiological signal transmitted by a nerve or emit an electrical pulse to the nerve. For example, the housing of the body may be configured as a counter electrode, and one or more electrodes on the helical nerve cuff may be configured as an operating electrode. The housing is made from a bioinert material, such as a bioinert metal (e.g., steel or titanium) or a bioinert ceramic (e.g., titania or alumina). The housing is preferably hermetically sealed, which prevents body fluids from entering the body.

An acoustic window can be included in the housing of the body, for example on the top of the housing (see FIG. 10). An acoustic window is a thinner material (such as a foil) that allows acoustic waves to penetrate the housing so that they may be received by one or more ultrasonic transducers within the body of the implantable device. In some embodiments, the housing (or the acoustic window of the housing) may be thin to allow ultrasonic waves to penetrate through the housing is about 100 micrometers ($\mu$m) or less in thickness, such as about 75 µm or less, about 50 µm or less, about 25 µm or less, about 15 µm or less, or about 10 µm or less. In some embodiments, the thickness of the housing (or the acoustic window of the housing) is about 5 µm to about 10 µm, about 10 µm to about 15 µm, about 15 µm to about 25 µm, about 25 µm to about 50 µm, about 50 µm to about 75 µm, or about 75 µm to about 100 µm in thickness.

The housing may be filled with an acoustically conductive material, such as a polymer or oil (such as a silicone oil). The material can fill empty space within the housing to reduce acoustic impedance mismatch between the tissue outside of the housing and within the housing. Accordingly, the body of the device is preferably void of air or vacuum. A port can be included on the housing, for example on the sidewall of the housing (see FIG. 10), to allow the housing to be filled with the acoustically conductive material. Once the housing is filled with the material, the port can be sealed to avoid leakage of the material after implantation.

The housing of the implantable device is relatively small, which allows for comfortable and long-term implantation while limiting tissue inflammation that is often associated with implantable devices. In some embodiments, the longest dimension of the housing of the device is about 8 mm or less, about 7 mm or less, about 6 m or less, about 5 mm or less, about 4 mm or less, about 3 mm or less, about 2 mm or less, about 1 mm or less, about 0.5 mm or less, about 0.3 mm or less, about 0.1 mm or less in length. In some embodiments, the longest dimension of the housing of the device is about 0.05 mm or longer, about 0.1 mm or longer, about 0.3 mm or longer, about 0.5 mm or longer, about 1 mm or longer, about 2 mm or longer, about 3 mm or longer, about 4 mm or longer, about 5 mm or longer, about 6 mm or longer, or about 7 mm or longer in the longest dimension of the device. In some embodiments, the longest dimension of the housing of the device is about 0.3 mm to about 8 mm in length, about 1 mm to about 7 mm in length, about 2 mm to about 6 mm in length, or about 3 mm to about 5 mm in length. In some embodiments, the housing of the implantable device has a volume of about 10 mm$^3$ or less (such as about 8 mm$^3$ or less, 6 mm$^3$ or less, 4 mm$^3$ or less, or 3 mm$^3$ or less). In some embodiments, the housing of the implantable device has a volume of about 0.5 mm$^3$ to about 8 mm$^3$, about 1 mm$^3$ to about 7 mm$^3$, about 2 mm$^3$ to about 6 mm$^3$, or about 3 mm$^3$ to about 5 mm$^3$.

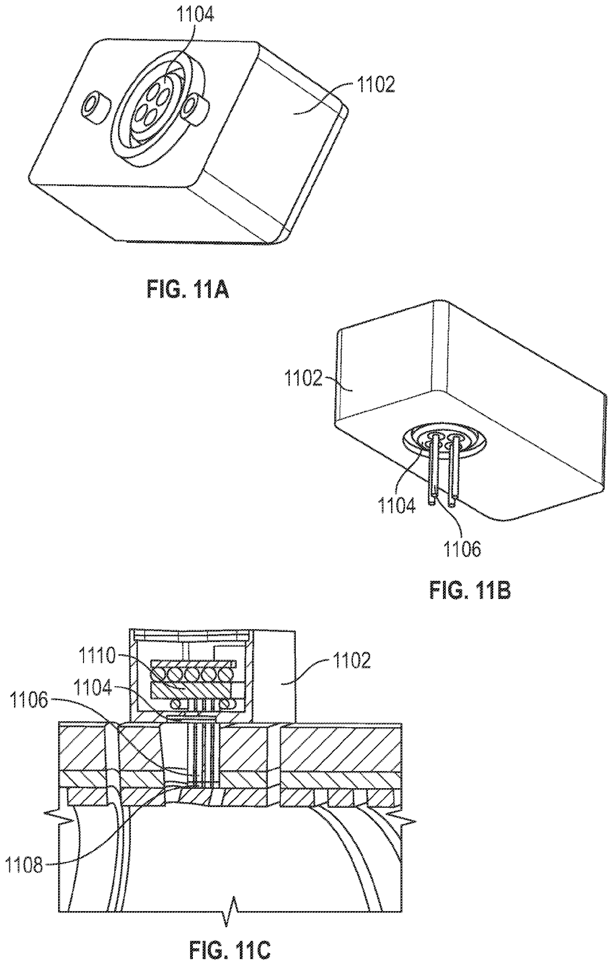

The housing (such as the bottom of the housing) can include a feedthrough port, which may be aligned with the feedthrough port of the nerve cuff. A feedthrough can electrically connect the one or more electrodes of the nerve cuff to components of the body within the housing. For example, the feedthrough may be electrically connected to an integrated circuit and/or the wireless communication system of the device body. FIG 11A shows a housing 1102 with a feedthrough port 1104, and FIG. 11B shows the housing with the feedthrough 1106 positioned to electrically connect the body components to one or more electrodes of the helical nerve cuff. FIG.

11C shows a cross sectional view of an exemplary device, wherein the feedthrough 1106 electronically connects electrodes 1108 on the nerve cuff to the electronic circuitry 1110 (integrated circuit, wireless communication system, etc.) positioned within the body housing 1102. The feedthroughs may be, for example, a metal (such as a metal comprising silver, copper, gold, platinum, platinum-black, or nickel) sapphire, or a conductive ceramic (for example indium tin oxide (ITO)). The electrodes may be connected to the feedthrough using any suitable means, such as soldering, laser welding, or crimping the feedthrough to the electrodes.

In some embodiments, the implantable device is implanted in a subject. The subject can be for example, a mammal. In some embodiments, the subject is a human, dog, cat, horse, cow, pig, sheep, goat, monkey, or a rodent (such as a rat or mouse). The helical nerve cuff may be configured to at least partially wrap around a filamentous tissue (such as a peripheral nerve, or a filamentous tissue comprising a peripheral nerve, such as a neurovascular bundle) within any of these animals, or others. For example, in some embodiments, the helical nerve cuff is configured to at least partially wrap around a human peripheral nerve, such as a human splenic nerve, or a human splenic neurovascular bundle. In some embodiments, the helical nerve cuff is configured to at least partially wrap around an autonomic nerve. In some embodiments, the nerve is a sympathetic nerve. In some embodiments, the nerve is a vagus nerve, a mesenteric nerve, a splenic nerve, a sciatic nerve, a tibial nerve, a pudendal nerve, a celiac ganglion, a sacral nerve, or any branch thereof.

**Interrogator**

The interrogator can wirelessly communicate with one or more implantable devices using the wireless communication system of the implantable device. Wireless communication may include wirelessly receiving information from the implantable device, wirelessly transmitting instructions to the implantable device (such as a trigger signal instructing the implantable device to emit an electrical pulse), or both. For example, in some embodiments, the interrogator transmits ultrasonic waves that encode instructions for operating the device, such as a trigger signal that instructs the implantable device to emit an electrical pulse. In some embodiments, the interrogator further receives ultrasonic backscatter from the implantable device, which encodes information transmitted by the implantable device. The information may include, for example, information related to a detected electrophysiological pulse, an electrical pulse emitted by the implantable device, and/or a measured physiological condition.

In some embodiments, the interrogator wirelessly transmits energy (such as ultrasonic waves or radiofrequency waves) to power the implantable device. For example, the interrogator may transmit ultrasonic waves or RF waves to the implantable device, which can convert energy from the received waves into an electrical energy that is used to power the device, either immediately or by storing the energy in a power circuit.

The interrogator may include one or more ultrasonic transducers or radiofrequency antennas (depending, for example, if the implantable device includes an ultrasonic transducer or radiofrequency antenna in its wireless communication system), which can operate as a transmitter and/or a receiver (or as a transceiver, which can be configured to alternatively transmit or receive the ultrasonic waves). The one or more transducers can be arranged as an array (e.g. a transducer array), and the interrogator can optionally include one or more arrays. In some embodiments, the transmitting function is separated from the receiving function on separate devices. That is, optionally, the interrogator comprises a first device that transmits ultrasonic or RF waves to the implantable device, and a second device that receives ultrasonic or RF backscatter from the implantable device. In some embodiments, the transducers or RF antennas in the array can have regular spacing, irregular spacing, or be sparsely placed. In some embodiments the array is flexible. In some embodiments the array is planar, and in some embodiments the array is non-planar.

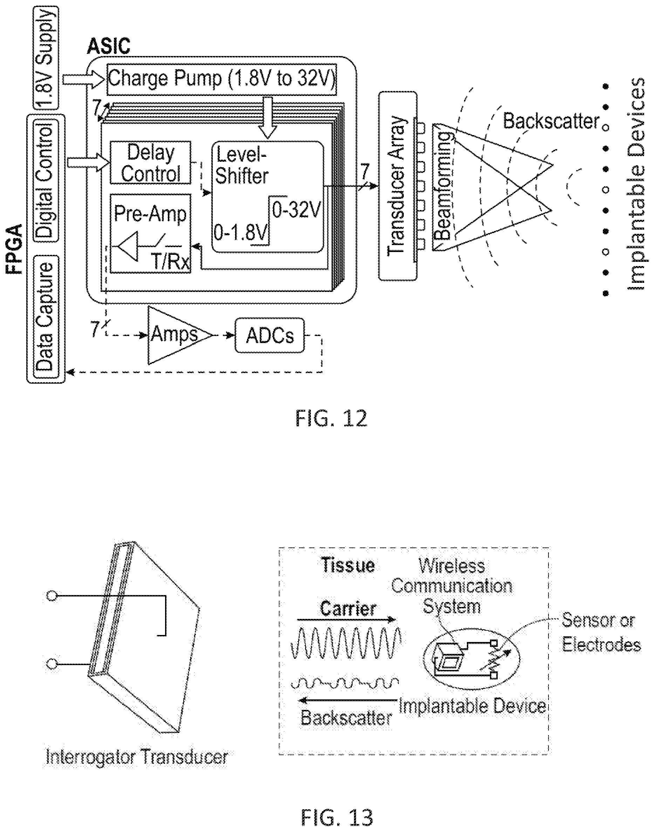

An exemplary interrogator is shown in FIG. 12. The illustrated interrogator shows a transducer array with a plurality of ultrasonic transducers. In some embodiments, the transducer array includes 1 or more, 2 or more, 3 or more, 5 or more, 7 or more, 10 or more, 15 or more, 20 or more, 25 or more, 50 or more, 100 or more 250 or more, 500 or more, 1000 or more, 2500 or more, 5000 or more, or 10,000 or more transducers. In some embodiments, the transducer array includes 100,000 or fewer, 50,000 or fewer, 25,000 or fewer, 10,000 or fewer, 5000 or fewer, 2500 or fewer, 1000 or fewer, 500 or fewer, 200 or fewer, 150 or fewer, 100 or fewer, 90 or fewer, 80 or fewer, 70 or fewer, 60 or fewer, 50 or fewer, 40 or fewer, 30 or fewer, 25 or fewer, 20 or fewer, 15 or fewer, 10 or fewer, 7 or fewer or 5 or fewer transducers. The transducer array can be, for example a chip comprising 50 or more ultrasonic transducer pixels.

The interrogator shown in FIG. 12 illustrates a single transducer array; however the interrogator can include 1 or more, 2 or more, or 3 or more separate arrays. In some embodiments, the interrogator includes 10 or fewer transducer arrays (such as 9, 8, 7, 6, 5, 4, 3, 2, or 1 transducer arrays). The separate arrays, for example, can be placed at different points of a subject, and can communicate to the same or different implantable devices. In some embodiments, the arrays are located on opposite sides of an implantable device. The interrogator can include an application specific integrated circuit (ASIC), which includes a channel for each transducer in the transducer array. In some embodiments, the channel includes a switch (indicated in FIG. 10 by "T/Rx"). The switch can alternatively configure the transducer connected to the channel to transmit ultrasonic waves or receive ultrasonic waves. The switch can isolate the ultrasound receiving circuit from the higher voltage ultrasound transmitting circuit.

In some embodiments, the transducer connected to the channel is configured only to receive or only to transmit ultrasonic waves, and the switch is optionally omitted from the channel. The channel can include a delay control, which operates to control the transmitted ultrasonic waves. The delay control can control, for example, the phase shift, time delay, pulse frequency and/or wave shape (including amplitude and wavelength). The delay control can be connected to a level shifter, which shifts input pulses from the delay control to a higher voltage used by the transducer to transmit the ultrasonic waves. In some embodiments, the data representing the wave shape and frequency for each channel can be stored in a 'wave table'. This allows the transmit waveform on each channel to be different. Then, delay control and level shifters can be used to 'stream' out this data to the actual transmit signals to the transducer array. In some embodiments, the transmit waveform for each channel can be produced directly by a high-speed serial output of a microcontroller or other digital system and sent to the transducer element through a level shifter or high-voltage amplifier. In some embodiments, the ASIC includes a charge pump (illustrated in FIG. 12) to convert a first voltage supplied to the ASIC to a higher second voltage, which is applied to the channel. The channels can be controlled by a controller, such as a digital controller, which operates the delay control.

In the ultrasound receiving circuit, the received ultrasonic waves are converted to current by the transducers (set in a receiving mode), which is transmitted to a data capture circuit. In some embodiments, an amplifier, an analog-to-digital converter (ADC), a variable-gain-amplifier, or a time-gain-controlled variable-gain-amplifier which compensates for tissue loss, and/or a band pass filter is included in the receiving circuit. The ASIC can draw power from a power supply, such as a battery (which is preferred for a wearable embodiment of the interrogator). In the embodiment illustrated in FIG. 12, a 1.8V supply is provided to the ASIC, which is increased by the charge pump to 32V, although any suitable voltage can be used. In some embodiments, the interrogator includes a processor and or a non-transitory computer readable memory. In some embodiments, the channel described above does not include a T/Rx switch but instead contains independent Tx (transmit) and Rx (receive) with a high-voltage Rx (receiver circuit) in the form of a low noise amplifier with good saturation recovery. In some embodiments, the T/Rx circuit includes a circulator. In some embodiments, the transducer array contains more transducer elements than processing channels in the interrogator transmit /receive circuitry, with a multiplexer choosing different sets of transmitting elements for each pulse. For example, 64 transmit receive channels connected via a 3:1 multiplexer to 192 physical transducer elements - with only 64 transducer elements active on a given pulse.

In some embodiments, the interrogator is implantable. In some embodiments, the interrogator is external (i.e., not implanted). By way of example, the external interrogator can be a wearable, which may be fixed to the body by a strap or adhesive. In another example, the external interrogator can be a wand, which may be held by a user (such as a healthcare professional). In some embodiments, the interrogator can be held to the body via suture, simple surface tension, a clothing-based fixation device such as a cloth wrap, a sleeve, an elastic band, or by sub-cutaneous fixation. The transducer or transducer array of the interrogator may be positioned separately from the rest of the transducer. For example, the transducer array can be fixed to the skin of a subject at a first location (such as proximal to one or more implanted devices), and the rest of the interrogator may be located at a second location, with a wire tethering the transducer or transducer array to the rest of the interrogator.

The specific design of the transducer array depends on the desired penetration depth, aperture size, and size of the individual transducers within the array. The Rayleigh distance, R, of the transducer array is computed as:

$$R = \frac{D^2 - \lambda^2}{4\lambda} \approx \frac{D^2}{4\lambda}, D^2 \gg \lambda^2$$

where D is the size of the aperture and $\lambda$ is the wavelength of ultrasound in the propagation medium (i.e., the tissue). As understood in the art, the Rayleigh distance is the distance at which the beam radiated by the array is fully formed. That is, the pressure field converges to a natural focus at the Rayleigh distance in order to maximize the received power. Therefore, in some embodiments, the implantable device is approximately the same distance from the transducer array as the Rayleigh distance.

The individual transducers in a transducer array can be modulated to control the Raleigh distance and the position of the beam of ultrasonic waves emitted by the transducer array through a process of beamforming or beam steering. Techniques such as linearly constrained minimum variance (LCMV) beamforming can be used to communicate a plurality of implantable devices with an external ultrasonic transceiver. See, for example, Bertrand et al., Beamforming Approaches for Untethered, Ultrasonic Neural Dust Motes for Cortical Recording: a Simulation Study, IEEE EMBC (Aug. 2014). In some embodiments, beam steering is performed by adjusting the power or phase of the ultrasonic waves emitted by the transducers in an array.

In some embodiments, the interrogator includes one or more of instructions for beam steering ultrasonic waves using one or more transducers, instructions for determining the relative location of one or more implantable devices, instructions for monitoring the relative movement of one or more implantable devices, instructions for recording the relative movement of one or more implantable devices, and instructions for deconvoluting backscatter from a plurality of implantable devices.

Optionally, the interrogator is controlled using a separate computer system, such as a mobile device (e.g., a smartphone or a tablet). The computer system can wirelessly communicate to the interrogator, for example through a network connection, a radiofrequency (RF) connection, or Bluetooth. The computer system may, for example, turn on or off the interrogator or analyze information encoded in ultrasonic waves received by the interrogator.

## Communication between an Implantable Device and an Interrogator

The implantable device and the interrogator wirelessly communicate with each other, for example using ultrasonic waves or radiofrequency waves. The communication may be a one-way communication (for example, the interrogator transmitting information to the implantable device, or the implantable device transmitting information to the interrogator), or a two-way communication (for example, the interrogator transmitting information to the implantable device, or the implantable device transmitting information to the interrogator). Information transmitted from the implantable device to the interrogator may rely on, for example, a backscatter communication protocol. For example, the interrogator may transmit ultrasonic or RF waves to the implantable device, which emits backscatter waves that encode the information. The interrogator can receive the backscatter waves and decipher the information encoded in the received backscatter waves.

In some embodiments, the implantable device receives ultrasonic or radiofrequency waves from the interrogator through one or more ultrasonic transducers or RF antennas on the implantable device, and the received waves can encode instructions for operating the implantable device. For example, vibrations of the ultrasonic transducer(s) on the implantable device generate a voltage across the electric terminals of the transducer, and current flows through the device, including the integrated circuit. The current (which may be generated, for example, using one or more ultrasonic transducers or RF antennas) can be used to charge an energy storage circuit, which can store energy to be used to emit an electrical pulse, for example after receiving a trigger signal. The trigger signal can be transmitted from the interrogator to the implantable device, signaling that an electrical pulse should be emitted. In some embodiments, the trigger signal includes information regarding the electrical pulse to be emitted, such as frequency, amplitude, pulse length, or pulse shape (e.g., alternating current, direct current, or pulse pattern). A digital circuit can decipher the trigger signal and operate the electrodes and electrical storage circuit to emit the pulse.

In some embodiments, ultrasonic backscatter or radiofrequency backscatter is emitted from the implantable device, which can encode information relating to the implantable device, the electrical pulse emitted by the implantable device, an electrophysiological pulse detected by the implantable device, or a detected physiological condition. For example, the backscatter can encode a verification signal, which verifies that electrical pulse was emitted. In some embodiments, an implantable device is configured to detect an electrophysiological signal, and information regarding the detected electrophysiological signal can be transmitted to the interrogator by the ultrasonic backscatter. To encode signals in the backscatter, current flowing through the ultrasonic transducer(s) of the implantable device is modulated as a function of the encoded information, such as a detected electrophysiological signal or measured physiological condition. In some embodiments, modulation of the current can be an analog signal, which may be, for example, directly modulated by the detected electrophysiological signal. In some embodiments, modulation of the current encodes a digitized signal, which may be controlled by a digital circuit in the integrated circuit. The backscatter is received by an external interrogator (which may be the same or different from the external interrogator that transmitted the initial ultrasonic waves). The information from the electrophysiological signal can thus be encoded by changes in amplitude, frequency, or phase of the backscattered ultrasound waves.

FIG. 13 shows an interrogator in communication with an implantable device. The external ultrasonic transceiver emits ultrasonic waves ("carrier waves"), which can pass through tissue. The carrier waves cause mechanical vibrations on the ultrasonic transducer (e.g., a bulk piezoelectric transducer, a PUMT, or a CMUT). A voltage across the ultrasonic transducer is generated, which imparts a current flowing through an integrated circuit on the implantable device. The current flowing through to the ultrasonic transducer causes the transducer on the implantable device to emit backscatter ultrasonic waves. In some embodiments, the integrated circuit modulates the current flowing through the ultrasonic transducer to encode information, and the resulting ultrasonic backscatter waves encode the information. The backscatter waves can be detected by the interrogator, and can be analyzed to interpret information encoded in the ultrasonic backscatter.

Communication between the interrogator and the implantable device can use a pulse-echo method of transmitting and receiving ultrasonic waves or RF waves. In the pulse-echo method, the interrogator transmits a series of interrogation pulses at a predetermined frequency, and then receives backscatter echoes from the implanted device. In some embodiments, the pulses are square, rectangular, triangular, sawtooth, or sinusoidal. In some embodiments, the pulses output can be two-level (GND and POS), three-level (GND, NEG, POS), 5-level, or any other multiple-level (for example, if using 24-bit DAC). In some embodiments, the pulses are continuously transmitted by the interrogator during operation. In some embodiments, when the pulses are continuously transmitted by the interrogator a portion of the transducers on the interrogator are configured to receive ultrasonic waves and a portion of the transducers on the interrogator are configured to transmit ultrasonic waves. Transducers configured to receive ultrasonic waves and transducers configured to transmit ultrasonic waves can be on the same transducer array or on different transducer arrays of the interrogator. In some embodiments, a transducer on the interrogator can be configured to alternatively transmit or receive the ultrasonic waves. For example, a transducer can cycle between transmitting one or more pulses and a pause period. The transducer is configured to transmit the ultrasonic waves when transmitting the one or more pulses, and can then switch to a receiving mode during the pause period.

In some embodiments, the backscattered waves are digitized by the implantable device. For example, the implantable device can include an oscilloscope or analog-to-digital converter (ADC) and/or a memory, which can digitally encode information in current (or impedance) fluctuations. The digitized current fluctuations, which can encode information, are received by wireless communication system, which then transmits digitized ultrasonic or radiofrequency waves. The digitized data can compress the analog data, for example by using singular value decomposition (SVD) and least squares-based compression. In some embodiments, the compression is performed by a correlator or pattern detection algorithm. The backscatter signal may go through a series of non-linear transformation, such as 4th order Butterworth bandpass filter rectification integration of backscatter regions to generate a reconstruction data point at a single time instance. Such transformations can be done either in hardware (i.e., hard-coded) or in software.

In some embodiments, the digitized data can include a unique identifier. The unique identifier can be useful, for example, in a system comprising a plurality of implantable devices and/or an implantable device comprising a plurality of electrode pairs. For example, the unique identifier can identify the implantable device of origin when from a plurality of implantable devices, for example when transmitting information from the implantable device (such as a verification signal). In some embodiments, an implantable device comprises a plurality of electrode pairs, which may simultaneously or alternatively emit an electrical pulse by a single implantable device. Different pairs of electrodes, for example, can be configured to emit an electrical pulse in different tissues (e.g., different nerves or different muscles) or in different regions of the same tissue. The digitized circuit can encode a unique identifier to identify and/or verify which electrode pairs emitted the electrical pulse.

In some embodiments, the digitized signal compresses the size of the analog signal. The decreased size of the digitized signal can allow for more efficient reporting of information encoded in the backscatter. By compressing the size of the transmitted information through digitization, potentially overlapping signals can be accurately transmitted.

In some embodiments, an interrogator communicates with a plurality of implantable devices. This can be performed, for example, using multiple-input, multiple output (MIMO) system theory. For example, communication between the interrogator and the plurality of implantable devices using time division multiplexing, spatial multiplexing, or frequency multiplexing. The interrogator can receive a combined backscatter from the plurality of the implantable devices, which can be deconvoluted, thereby extracting information from each implantable device. In some embodiments, interrogator focuses the ultrasonic waves transmitted from a transducer array to a particular implantable device through beamsteering. The interrogator focuses the transmitted ultrasonic waves to a first implantable device, receives backscatter from the first implantable device, focuses transmitted ultrasonic waves to a second implantable device, and receives backscatter from the second implantable device. In some embodiments, the interrogator transmits ultrasonic waves to a plurality of implantable devices, and then receives ultrasonic waves from the plurality of implantable devices.

**Methods of Implanting the Nerve Cuff and Device**

The nerve cuff, or the implantable device comprising the nerve cuff, is implanted in a subject such that the helical nerve cuff at least partially wraps around a filamentous tissue comprising a nerve, which positions the electrodes of the device in electrical communication with the nerve. The nerve cuff or implantable device having the nerve cuff may be implanted in a surgical process using available surgical tools. Certain nerves or neurovascular bundles within a subject are limiting in space, and the implantation methods described herein may be beneficial for implanting the device or helical nerve cuff within a small surgical field.

The implantable device or helical nerve cuff may be implanted in the subject by at least partially unwinding the helical nerve cuff, passing an end of the helical nerve cuff behind a fibrous tissue comprising a nerve, and pulling the end of the helical nerve cuff that passed behind the fibrous tissue. Partially unwinding the helical nerve cuff configures the nerve cuff in a flexed state, and the nerve cuff may be released into a relax state, which wraps the helical nerve cuff around the fibrous tissue. The helical nerve cuff may be positioned once wrapped around the helical nerve cuff, for example by tucking the end of the helical nerve cuff under or behind the fibrous tissue.

The fibrous tissue may be isolated for implantation by separating the fibrous tissue from surrounding tissue, for example by circumferentially dissecting or partially circumferentially dissecting a portion of the fibrous tissue. An initial incision may be made to access the fibrous tissue, which may be overlaid by other tissue. Once the fibrous tissue is isolated, the implantable device or helical nerve cuff may be brought into the surgical field. The helical nerve cuff or implantable device may be oriented such that the axis of the helical nerve cuff is substantially parallel to the fibrous tissue. In this orientation, an end of the helical nerve cuff may be passed behind the fibrous tissue (for example from underneath the fibrous tissue).

The end of the helical nerve cuff may be initially threaded behind the fibrous tissue by pushing the end of the helical nerve cuff. A handle portion (e.g., a filament, suture, loop, etc.) may be attached to the end of the helical nerve cuff, which can be pulled to thread the helical nerve cuff behind the filamentous tissue.

In some embodiments, the helical nerve cuff is at least partially unwound prior to pulling the end of the helical nerve cuff passed behind the fibrous tissue. For example, a handle portion at one or both ends of the helical nerve cuff may be grasped and the helical nerve cuff manipulated to unwind the helix in a radial movement. Unwinding the helical nerve cuff configures the nerve cuff in a flexed configuration, and the nerve cuff can recoil in a relaxed configuration to rewind the helix. The end of the at least partially unwound helix can then be passed behind the fibrous tissue (which may include pulling on the end of the helical nerve cuff). Once the end is passed, the flexed helical nerve cuff may be released, which cases the helical nerve cuff to wrap around the fibrous tissue. The helical nerve cuff (and/or body, if present) can then be repositioned to ensure contact between the electrodes of the helical nerve cuff and the fibrous tissue.

In some embodiments, the helical nerve cuff is at least partially unwound by pulling the end of the helical nerve cuff passed behind the fibrous tissue. For example, the end of the helical nerve cuff may be passed behind the fibrous tissue with the helical nerve cuff in a relaxed configuration (i.e., a wound helix). The end of the helical nerve cuff passed behind the fibrous tissue can then be pulled (for example, by pulling a handle portion attached to the end of the helical nerve cuff). Pulling the end of the helical nerve cuff causes the nerve cuff to partially unwind as it is passed behind the filamentous tissue. The helical nerve cuff (and/or body, if present) can then be repositioned to ensure contact between the electrodes of the helical nerve cuff and the fibrous tissue.

The body of the implantable device may be attached to a first end of the helical nerve cuff or the middle of the helical nerve cuff, leaving the second end of the helical nerve cuff configured to pass behind the fibrous tissue. Optionally, the body may be held in an approximately steady position as the end of the helical nerve cuff is passed behind the fibrous tissue. For example, the implantable device may include a handle portion attached to the helical nerve cuff at a position proximal to the body or attached to the body itself, and this handle portion may be grasped to stabilize the body. There may be some slight movement of the body, but movement of the body can be limited to avoid the body catching on surrounding tissue or the fibrous tissue when the helical nerve cuff is passed behind the fibrous tissue. Nevertheless, in some embodiments, the body of the implantable device is unrestrained, and may freely move as the helical nerve cuff is passed behind the fibrous tissue.

Once the helical nerve cuff of an implantable devices is wrapped around the filamentous tissue, the body of the implantable device may be positioned in the desired orientation. For example, if the body includes an acoustic window, the acoustic window of the body can be oriented in a direction of a desired interrogator position.

The implantable device is implanted in a mammalian subject. In some embodiments, the subject is a human, dog, cat, horse, cow, pig, sheep, goat, monkey, or a rodent (such as a rat or mouse). The helical nerve cuff may implanted such that it at least partially wrap around a filamentous tissue (such as a peripheral nerve, or a filamentous tissue comprising a peripheral nerve, such as a neurovascular bundle) within any of these animals, or others. For example, in some embodiments, the helical nerve cuff or implantable device is implanted such that the nerve cuff at least partially wrap around a human peripheral nerve, such as a human splenic nerve, or a human splenic neurovascular bundle. In some embodiments, the helical nerve cuff or implantable device is implanted such that the nerve cuff at least partially wrap around an autonomic nerve. In some embodiments, the nerve is a sympathetic nerve. In some embodiments, the nerve is a vagus nerve, a mesenteric nerve, a splenic nerve, a sciatic nerve, a tibial nerve, a pudendal nerve, a celiac ganglion, a sacral nerve, or any branch thereof.

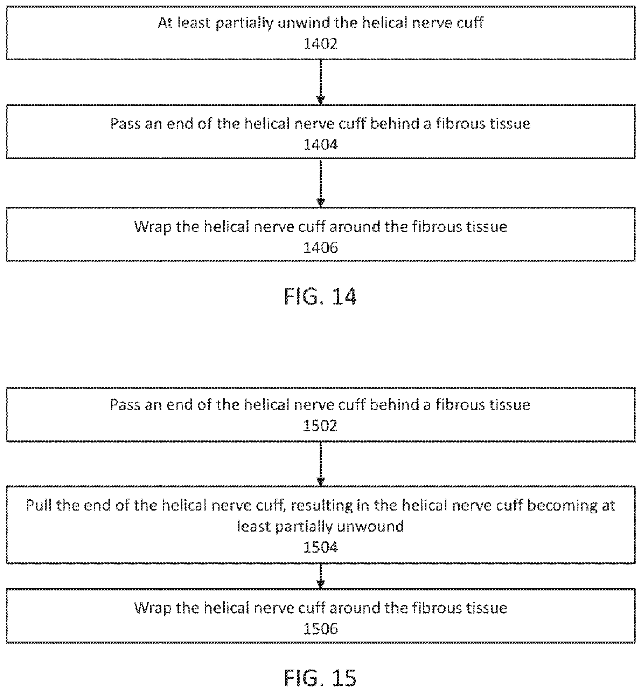

FIG. 14 is a flowchart demonstrating a method of implanting a helical nerve cuff in a subject. At step 1402, the helical nerve cuff is at least partially unwound, which configures the helical nerve cuff in a flexed configuration. At step 1404, an end of the helical nerve cuff is passed behind a fibrous tissue comprising a nerve. The fibrous tissue may be, for example, neurovascular bundle, such as splenic nerve and splenic artery. The end of the helical nerve cuff may be pushed underneath the fibrous tissue, which exposes a handle portion on the opposite side of the filamentous tissue. Optionally, the handle portion may be pulled to pass the helical nerve cuff behind the fibrous tissue. At step 1406, the helical nerve cuff is wrapped around the fibrous tissue. Releasing the helical nerve cuff from flexed configuration allows the nerve cuff to recoil into a relaxed configuration, which may wrap the helical nerve cuff around the fibrous tissue. Optionally, the helical nerve cuff may be repositioned (for example by pulling on one or more handle portions) to ensure the one or more electrodes of the helical nerve cuff contact the fibrous tissue.

FIG. 15 is a flowchart demonstrating another method of implanting a helical nerve cuff in a subject. At step 1502, an end of the helical nerve cuff is passed behind a fibrous tissue comprising a nerve. The fibrous tissue may be, for example, neurovascular bundle, such as splenic nerve and splenic artery. The end of the helical nerve cuff may be pushed underneath the fibrous tissue, which exposes a handle portion on the opposite side of the filamentous tissue. At step 1504, the end of the helical nerve cuff is pulled (for example by a handled) to pass the helical nerve cuff behind the fibrous tissue, which causes the helical nerve cuff to become at least partially unwound. At step 1506, the helical nerve cuff is wrapped around the fibrous tissue. Releasing the helical nerve cuff from flexed configuration allows the nerve cuff to recoil into a relaxed configuration, which may wrap the helical nerve cuff around the fibrous tissue. Optionally, the helical nerve cuff may be repositioned (for example by pulling on one or more handle portions) to ensure the one or more electrodes of the helical nerve cuff contact the fibrous tissue.

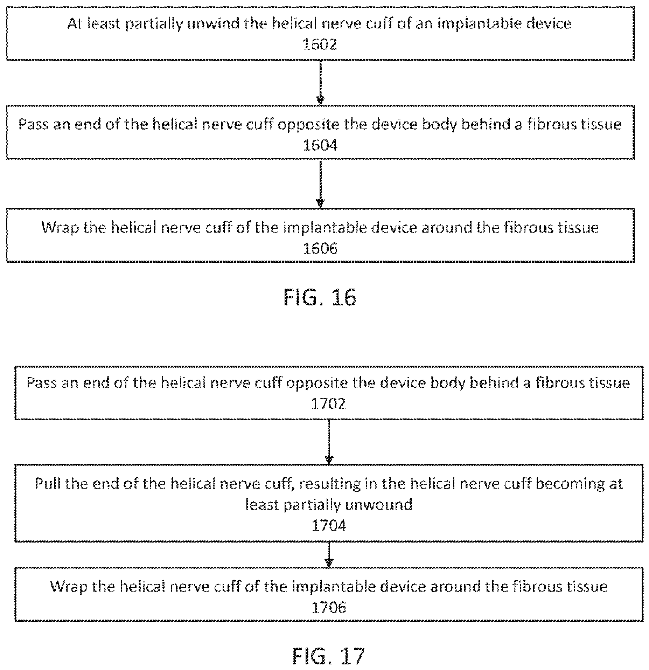

FIG. 16 is a flowchart demonstrating a method of implanting an implantable device comprising a helical nerve cuff in a subject. The body of the implantable device is attached to a first end of the helical nerve cuff. At step 1602, the helical nerve cuff of the implantable device is at least partially unwound, which configures the helical nerve cuff in a flexed configuration. At step 1604, a second end of the helical nerve cuff is passed behind a fibrous tissue comprising a nerve. The fibrous tissue may be, for example, neurovascular bundle, such as splenic nerve and splenic artery. The second end of the helical nerve cuff may be pushed underneath the fibrous tissue, which exposes a handle portion on the opposite side of the filamentous tissue. Optionally, a handle portion attached to the helical nerve cuff proximal to the second end may be pulled to pass the helical nerve cuff behind the fibrous tissue. At step 1606, the helical nerve cuff is wrapped around the fibrous tissue. Releasing the helical nerve cuff from flexed configuration allows the nerve cuff to recoil into a relaxed configuration, which may wrap the helical nerve cuff around the fibrous tissue. Optionally, the helical nerve cuff and/or the body of the implantable device may be repositioned (for example by pulling on one or more handle portions) to ensure the one or more electrodes of the helical nerve cuff contact the fibrous tissue.

FIG. 17 is a flowchart demonstrating another method of implanting an implantable device comprising a helical nerve cuff in a subject. The body of the implantable device is attached to a first end of the helical nerve cuff. At step 1702, a second end of the helical nerve cuff is passed behind a fibrous tissue comprising a nerve. The fibrous tissue may be, for example, neurovascular bundle, such as splenic nerve and splenic artery. The end of the helical nerve cuff may be pushed underneath the fibrous tissue, which exposes a handle portion on the opposite side of the filamentous tissue. At step 1704, the second end of the helical nerve cuff is pulled (for example by a handled) to pass the helical nerve cuff behind the fibrous tissue, which causes the helical nerve cuff to become at least partially unwound. At step 1706, the helical nerve cuff is wrapped around the fibrous tissue. Releasing the helical nerve cuff from flexed configuration allows the nerve cuff to recoil into a relaxed configuration, which may wrap the helical nerve cuff around the fibrous tissue. Optionally, the helical nerve cuff may be repositioned (for example by pulling on one or more handle portions) to ensure the one or more electrodes of the helical nerve cuff contact the fibrous tissue.

**Methods of Making the Implantable Device**

The implantable device can be made by attaching a device body to a helical nerve cuff, or by assembling the helical nerve cuff on the device body. For example, the helical nerve cuff may comprise two or more substrate layers, and a first substrate layer may be attached to the device body before a second substrate layer is attached to the first substrate layer, thus forming the helical nerve cuff.

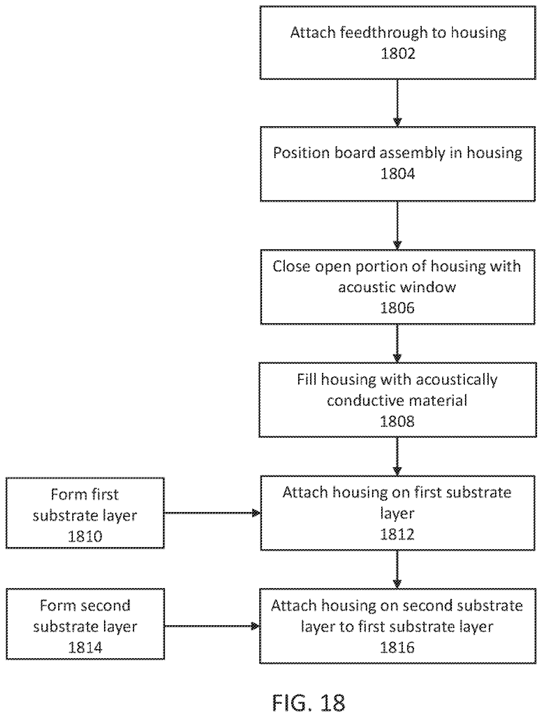

FIG. 18 shows a flowchart of an exemplary method of making an implantable device. Although the steps of the method are discussed in an exemplary order, it is understood that the order of the steps may be changed as would be appreciated by one skilled in the art.

At step 1802, a feedthrough is attached to a housing. The housing can include a feedthrough port, for example on the bottom of the housing, which is configured to receive the feedthrough. The feedthrough may then be attached to the housing at the feedthrough port, for example by welding or braising the feedthrough to the housing. Attaching the feedthrough to the feedthrough port of the housing can create a seal at the feedthrough port.

At step 1804, a board assembly is positioned within the housing. The board assembly may include a wireless communication system (e.g., one or more ultrasonic transducers), an integrated circuit, and/or other electronic components of the body. One or more of these components may be attached to a flex board, for example by using a conductive adhesive and/or wirebonding the components to form the board assembly. The board assembly is positioned within the housing to electrically connect the feedthrough to the board assembly. Alternatively, the feedthrough may be attached to the board assembly before the board assembly is positioned in the housing, and the feedthrough can then be attached to the housing once the board assembly is in place.

At step 1806, an open portion of the housing is closed with a lid, for example an acoustic window. The board assembly may be positioned in the housing, for example, through the open portion. The lid may include an acoustic window. For example, if the wireless communication system of the board assembly includes one or more ultrasonic transducers, an acoustic window can be included to allow ultrasonic waves to penetrate the housing. An acoustic window may be formed, for example, by attaching a foil to a frame (for example by diffusion bonding the foil to the frame). Then, the acoustic window may be attached to the open housing, for example by laser welding.

At step 1808, an acoustically conductive material is filled into the housing. This step is optional, and may not be applicable for example if the wireless communication system is not an acoustically-based communication system. However, if the wireless communication system includes one or more ultrasonic transducers, filling the housing with an acoustically conductive material can allow for better communication using ultrasonic waves. The acoustically conductive material may be filled in the housing using a port, which may be included on the side of the housing. A vacuum may optionally be used to ensure no bubbles are present in the acoustically conductive material. Once the housing is filled, the port can be sealed, for example by placing a plug in the port, which may be soldered or laser welded to seal the plug is position.

Once assembled, the housing is preferably hermetically sealed (for example, by sealing any housing opening, such as an open housing top, the feedthrough port and/or the acoustically conductive material port).

At step 1810, a first substrate layer for the nerve cuff is formed. The first nerve cuff layer may be molded or cut into a helical shape. A feedthrough port may also be cut or molded into the first nerve cuff layer, which is configured to allow the feedthrough attached to the housing to pass through the feedthrough port of the first layer.

At step 1812, the first substrate layer (i.e., the outer layer) is attached to the housing. The housing is aligned with the first substrate layer such that the feedthrough attached to the housing passes through the feedthrough port of the first substrate layer. The housing may be positioned as desired relative to the helical axis, such as parallel to or perpendicular to the helical axis. An adhesive and/or one or more fasteners may be used to secure the housing to the first substrate layer. For example, the housing may have one or more fasteners attached (e.g., soldered, welded, of formed form the housing), which can be passed through the first substrate and manipulated to secure the housing to the first layer.

At step 1814, the second substrate layer (e.g., the inner layer) is formed. One or more electrodes may be shaped (for example, by laser cutting an electrically conductive material), and then embedded in a second substrate material. If needed, flashing may be removed from the second substrate material to ensure the desired expos

At step 1816, the second substrate layer is attached to the first substrate layer. The second substrate layer may be aligned with the first substrate layer to align the feedthrough with the electrodes, thereby electrically connecting the electrodes to the board assembly through the feedthrough. The electrodes may be attached to the feedthrough, for example by soldering the feedthrough to the electrodes and/or using an adhesive. An adhesive may then be used to attach the second substrate layer to the first substrate layer, thereby forming the helical nerve cuff. Optionally, the second substrate layer is wrapped around a mandrel, and the first substrate layer may be wrapped around the second substrate layer while the second substrate layer is wrapped around the mandrel. In some embodiments, one or more handle portions are attached to the device, for example by attaching the one or more handle portions to the housing, the first substrate layer, the second substrate layer, or between the first substrate layer and the second substrate layer when the first substrate layer is attached to the second substrate layer.

EXEMPLARY EMBODIMENTS

The following embodiments are exemplary and are not intended to limit the scope of the present application:

Embodiment 1. An implantable device, comprising:

a body comprising a wireless communication system;

two or more electrodes in electrical communication with the wireless communication system configured to detect an electrophysiological signal transmitted by a nerve or emit an electrical pulse to the nerve; and

a helical nerve cuff comprising at least one of the two or more electrodes, wherein the body is on the helical nerve cuff, and the helical nerve cuff is configured to at least partially wrap around a filamentous tissue comprising the nerve and position the at least one of the two or more electrodes in electrical communication with the nerve.

Embodiment 2. The implantable device of embodiment 1, wherein the wireless communication system comprises an ultrasonic transducer.

Embodiment 3. The implantable device of embodiment 2, wherein the ultrasonic transducer is about 5 mm or less in length in the longest dimension.

Embodiment 4. The implantable device of embodiment 2 or 3, wherein the wireless communication system comprises two or more ultrasonic transducers.

Embodiment 5. The implantable device of embodiment 1, wherein the wireless communication system comprises a radiofrequency antenna.

Embodiment 6. The implantable device of any one of embodiments 1-5, wherein the wireless communication system is configured to receive ultrasonic waves or radiofrequency waves, and convert energy from the ultrasonic waves or radiofrequency waves into an electrical energy that powers the device.

Embodiment 7. The implantable device of any one of embodiments 1-6, wherein the helical nerve cuff is configured to wrap around the nerve by at least one revolution.

Embodiment 8. The implantable device of embodiment 7, wherein the helical nerve cuff is configured to wrap around the nerve by about 1.3 to about 1.7 revolutions.

Embodiment 9. The implantable device of any one of embodiments 1-8, wherein the helical nerve cuff has a width that defines an inner surface, a first edge, and a second edges of the helical nerve cuff, and wherein at least a portion of the first edge contacts at least a portion of the second edge when the helical nerve cuff is in a relaxed position.

Embodiment 10. The implantable device of any one of embodiments 1-8, wherein the helical nerve cuff has a width that defines an inner surface, a first edge, and a second edges of the helical nerve cuff, and wherein the first edge does not contact the second edge when the helical nerve cuff is in a relaxed position.

Embodiment 11. The implantable device of any one of embodiments 1-10, wherein at least one of the two or more electrodes is positioned along the length of the helical nerve cuff.

Embodiment 12. The implantable device of embodiment 11, wherein the at least one of the two or more electrodes positioned along the length of the helical nerve cuff is positioned on an inner surface of the helical nerve cuff.

Embodiment 13. The implantable device of any one of embodiments 1-12, wherein the at least one electrode on the helical nerve cuff comprises one or more serpentine segments.

Embodiment 14. The implantable device of any one of embodiments 1-13, wherein the helical nerve cuff is flexible and is configurable in (a) a flexed position by at least partially unwinding the helical nerve cuff, and (b) a relaxed position.

Embodiment 15. The implantable device of any one of embodiments 1-14, further comprising a handle portion attached the helical nerve cuff or the body.

Embodiment 16. The implantable device of embodiment 15, wherein the handle portion comprises a loop.

Embodiment 17. The implantable device of embodiment 15 or 16, wherein the handle portion comprises a filament.

Embodiment 18. The implantable device of any one of embodiments 15-17, wherein the handle portion is attached to the nerve cuff at a position proximal to an end of the helical nerve cuff.

Embodiment 19. The implantable device of embodiment 18, further comprising a second handle portion attached to the helical nerve cuff at a position proximal to a second end of the helical nerve cuff.

Embodiment 20. The implantable device of embodiment 19, wherein the first handle portion is attached to the second handle.

Embodiment 21. The implantable device of any one of embodiments 16-20, further comprising an additional handle portion attached to a middle position along the length of the helical nerve cuff.

Embodiment 22. The implantable device of any one of embodiments 1-21, further comprising a mount configured to receive the body and the helical nerve cuff, thereby attaching the body on the helical nerve cuff.

Embodiment 23. The implantable device of any one of embodiments 1-21, wherein the body is directly attached on an outer surface of the helical nerve cuff.

Embodiment 24. The implantable device of any one of embodiments 1-23, wherein the body is attached to an end of the helical nerve cuff.

Embodiment 25. The implantable device any one of embodiments 1-23, wherein the body is attached to middle portion of the helical nerve cuff.

Embodiment 26. The implantable device of any one of embodiments 1-25, wherein the body comprises an attachment end attached to the helical nerve cuff and an extension end extending from the attachment end.

Embodiment 27. The implantable device of any one of embodiments 1-26, wherein the helical nerve cuff comprises a right-handed helical portion.

Embodiment 28. The implantable device of any one of embodiments 1-26, wherein the helical nerve cuff comprises a left-handed helical portion.

Embodiment 29. The implantable device of any one of embodiments 1-28, wherein the helical nerve cuff comprises a right-handed helical portion joined to a left-handed helical portion.

Embodiment 30. The implantable device of embodiment 29, wherein the body attaches to the helical nerve cuff at a position proximal to where the right-handed helix portion is joined to the left-handed helical portion.

Embodiment 31. The implantable device of embodiment 29 or 30, wherein the right-handed helical portion is joined to a left-handed helical portion through a linear joining portion.

Embodiment 32. The implantable device of any one of embodiments 1-31, wherein the helical nerve cuff comprises one or more electrodes configured to emit an electrical pulse to the nerve.

Embodiment 33. The implantable device of anyone of embodiments 1-32, wherein the helical nerve cuff comprises one or more electrodes configured to detect the electrophysiological signal transmitted by the nerve.

Embodiment 34. The implantable device of embodiments 33, wherein the helical nerve cuff comprises two or more electrodes configured to detect the electrophysiological signal transmitted by the nerve.

Embodiment 35A. The implantable device of any one of embodiments 1-34, wherein the nerve is a human splenic nerve.

Embodiment 35B. The implantable device of any one of embodiments 1-34, wherein the nerve is a human splanchnic nerve.

Embodiment 36. The implantable device of any one of embodiments 1-35A and 35B, wherein the body comprises a housing.

Embodiment 37. The implantable device of embodiment 36, wherein the housing is configured as one of the two or more electrodes.

Embodiment 38. The implantable device of embodiment 36 or 37, wherein the housing comprises an acoustic window.

Embodiment 39. The implantable device of any one of embodiments 36-38, wherein the housing contains an acoustically conductive material.

Embodiment 40. The implantable device of any one of embodiments 1-39, wherein the body comprises an integrated circuit electrically connected to the wireless communication system and the two or more electrodes.

Embodiment 41. The implantable device of embodiment 40, wherein the integrated circuit comprises an energy storage circuit comprising a capacitor.

Embodiment 42. The implantable device of any one of embodiments 1-41, wherein the body is about 8 mm or less in length in the longest dimension.

Embodiment 43. The implantable device of any one of embodiments 1-42, wherein the wireless communication system is configured to transmit data.

Embodiment 44. The implantable device of embodiment 43, wherein the wireless communication system is configured to emit an ultrasonic backscatter or radiofrequency backscatter that encodes the data.

Embodiment 45. The implantable device of embodiment 43 or 44, wherein the data comprises information related to a detected electrophysiological signal, a measured physiological condition, a device status, or an emitted electrical pulse.

Embodiment 46. The implantable device of any one of embodiments 1-45, wherein the wireless communication system is configured to receive instructions for operating the implantable device.

Embodiment 47. The implantable device of embodiment 46, wherein the instructions are encoded in ultrasonic waves or radiofrequency waves.

Embodiment 48. The implantable device of embodiment 46 or 47, wherein the instructions comprise a trigger signal that operates the implantable device to emit an electrical pulse to the nerve.

Embodiment 49. The implantable device of any one of embodiments 1-48, wherein the implantable device further comprises a sensor configured to detect a physiological condition.

Embodiment 50. The implantable device of embodiment 49, wherein the sensor is configured to detect temperature, pH, pressure, strain, or an analyte concentration.

Embodiment 51. The implantable device of any one of embodiments 1-50, wherein the fibrous tissue comprises a blood vessel.

Embodiment 52. A system, comprising the implantable device of any one of embodiments 1-51, and an interrogator comprising a second wireless communication system configured to wirelessly communicate with the wireless communication system of the implantable device.

Embodiment 53. The system of embodiments 52, wherein the second wireless communication system comprises one or more ultrasonic transducers configured to transmit ultrasonic waves to the implantable medical device, wherein the ultrasonic waves power the implantable medical device.

Embodiment 54. The system of embodiment 52, wherein the second wireless communication system comprises one or more radiofrequency antennas configured to transmit radiofrequency waves to the implantable medical device, wherein the radiofrequency waves power the implantable medical device.

Embodiment 55. The system of any one of embodiments 52-54, wherein the interrogator is configured to be worn externally.

Embodiment 56. A nerve cuff, comprising:

a flexible helical substrate configured to at least partially wrap around a filamentous tissue comprising a nerve, and is configurable in (a) a flexed position by at least partially unwinding the helical nerve cuff, and (b) a relaxed position;
one or more electrodes positioned along the length of the helical substrate; and
a handle portion attached to the helical substrate configured to apply a force that configures the helical substrate in the flexed position.

Embodiment 57. The nerve cuff of embodiment 56, wherein the handle portion comprises a loop.

Embodiment 58. The nerve cuff of embodiment 56 or 57, wherein the handle portion comprises a filament.

Embodiment 59. The nerve cuff of any one of embodiments 56-58, wherein the helical nerve cuff is configured to wrap around the nerve by at least one revolution.

Embodiment 60. The nerve cuff of embodiment 59, wherein the helical nerve cuff is configured to wrap around the nerve by about 1.3 to about 1.7 revolutions.

Embodiment 61. The nerve cuff of any one of embodiments 56-60, wherein a portion of the handle portion is embedded within the substrate.

Embodiment 62. The nerve cuff of any one of embodiments 56-61, wherein the handle portion is attached to the nerve cuff at a position proximal to an end of the helical nerve cuff.

Embodiment 63. The nerve cuff of embodiment 62, further comprising a second handle portion attached to the helical nerve cuff at a position proximal to a second end of the helical nerve cuff.

Embodiment 64. The nerve cuff of embodiment 63, wherein the first handle portion and the second handle portion are separated by a radial angle of about 90° to about 180° around a helical axis.

Embodiment 65. The nerve cuff of embodiment 63 or 64, wherein the first handle portion is attached to the second handle portion.

Embodiment 66. The implantable device of any one of embodiments 56-65, further comprising an additional handle portion attached to a middle position along the length of the helical nerve cuff.

Embodiment 67. The nerve cuff of any one of embodiments 56-66, wherein the substrate has a width that defines an inner surface, a first edge of the substrate, and a second edge of the substrate, and wherein at least a portion of the first edge contacts at least a portion of the second edge when the nerve cuff is in a relaxed position.

Embodiment 68. The nerve cuff of any one of embodiments 56-66, wherein the substrate has a width that defines an inner surface, a first edge of the substrate, and a second edge of the substrate, and wherein the first edge does not contact the second edge when the nerve cuff is in a relaxed position.

Embodiment 69. The nerve cuff of any one of embodiments 56-68, wherein the at least one of the one or more electrodes is positioned along the length of the helical nerve cuff.

Embodiment 70. The nerve cuff of embodiment 69, wherein the at least one of the one or more electrodes positioned along the length of the helical nerve cuff is positioned on an inner surface of the helical nerve cuff.

Embodiment 71. The nerve cuff of any one of embodiments 56-70, wherein the at least one of the one or more electrodes comprises one or more serpentine segments.

Embodiment 72. The nerve cuff of any one of embodiments 56-71, wherein the helical nerve cuff is configured to be unwound by at least one revolution.

Embodiment 73. The nerve cuff of any one of embodiments 56-72, wherein the helical nerve cuff comprises a right-handed helical portion.

Embodiment 74. The nerve cuff of any one of embodiments 56-72, wherein the helical nerve cuff comprises a left-handed helical portion.

Embodiment 75. The nerve cuff of any one of embodiments 56-73, wherein the helical nerve cuff comprises a right-handed helical portion joined to a left-handed helical portion.

Embodiment76. The nerve cuff of any one of embodiments 56-75, comprising a first helical portion joined to a second helical portion through a linear connecting member.

Embodiment 77. The nerve cuff of any one of embodiments 56-76, wherein the nerve cuff comprises one or more electrodes configured to emit an electrical pulse to the nerve.

Embodiment 78. The nerve cuff of anyone of embodiments 56-77, wherein the nerve cuff comprises one or more electrodes configured to detect the electrophysiological signal transmitted by the nerve.

Embodiment 79. The nerve cuff of embodiments 78, wherein the nerve cuff comprises two or more electrodes configured to detect the electrophysiological signal transmitted by the nerve.

Embodiment 80. The nerve cuff of any one of embodiments 56-79, wherein the nerve cuff comprises one or more electrodes configured to detect the electrophysiological signal transmitted by the nerve, and one or more electrodes configured to emit an electrical pulse to the nerve, wherein at least one of the one or more electrodes configured to emit an electrical pulse to the nerve is wider than at least one of the one or more electrodes configured to detect the electrophysiological signal transmitted by the nerve.

Embodiment 81. The nerve cuff of any one of embodiments 56-80, further comprising a mount attached to the helical substrate configured to receive a body and electrically connect the one or more electrodes positioned along the length of the helical substrate to the body.

Embodiment 82. The nerve cuff of embodiment 81, wherein the body directly connects with the one or more electrodes.

Embodiment 83. The nerve cuff of embodiment 81, wherein the mount comprises one or more feedthroughs configured to electrically connect the body to the one or more electrodes.

Embodiment 84A. The nerve cuff of any one of embodiments 56-83, wherein the nerve is a splenic nerve.

Embodiment 84B. The nerve cuff of any one of embodiments 56-83, wherein the nerve is a splanchnic nerve.

Embodiment 85. A method of implanting a helical nerve cuff comprising one or more electrodes, comprising:

> at least partially unwinding the helical nerve cuff;
> passing an end of the helical nerve cuff behind a fibrous tissue comprising a nerve; and
> wrapping the helical nerve cuff around the fibrous tissue.

Embodiment 86. The method of embodiment 85, wherein the helical nerve cuff is the nerve cuff of any one of embodiments 56-84A and 84B.

Embodiment 87. A method of implanting an implantable device comprising a body comprising a wireless communication system on a helical nerve cuff comprising one or more electrodes, the method comprising:

> at least partially unwinding the helical nerve cuff;
> passing an end of the helical nerve cuff behind a fibrous tissue comprising a nerve; and
> wrapping the helical nerve cuff around the fibrous tissue.

Embodiment 88. The method of embodiment 87, comprising limiting movement of the body as the end of the helical nerve cuff is passed behind the fibrous tissue.

Embodiment 89. The method of embodiment 88, wherein the body is held in an approximately steady position by holding a handle portion attached to the helical nerve cuff at a position proximal to the body or a handle portion attached to the body.

Embodiment 90. The method of any one of embodiments 87-89, wherein the implantable device is the implantable device of any one of embodiments 1-51.

Embodiment 91. The method of any one of embodiments 85-90, comprising pulling the end of the helical nerve cuff passed behind the fibrous tissue.

Embodiment 92. The method of any one of embodiments 85-91, comprising orienting the helical nerve cuff substantially parallel to the fibrous tissue.

Embodiment 93. The method of any one of embodiments 85-92, wherein the helical nerve cuff is at least partially unwound by pulling the end of the helical nerve cuff passed behind the fibrous tissue.

Embodiment 94. The method of any one of embodiments 85-92, wherein the helical nerve cuff is at least partially unwound prior to pulling the end of the helical nerve cuff passed behind the fibrous tissue.

Embodiment 95. The method of any one of embodiments 85-94, wherein the end of the helical nerve cuff is passed behind the fibrous tissue from underneath the fibrous tissue.

Embodiment 96. The method of any one of embodiments 85-95, further comprising separating the fibrous tissue from surrounding tissue.

Embodiment 97. The method of embodiment 96, wherein the fibrous tissue is separated from the surrounding tissue by circumferentially dissecting a portion of the fibrous tissue.

Embodiment 98. The method of any one of embodiment 85-97, wherein the end of the helical nerve cuff passed behind the fibrous tissue is pulled by pulling a handle portion attached to the end of the helical nerve cuff.

Embodiment 99A. The method of any one of embodiments 85-98, wherein the fibrous tissue comprises a splenic nerve.

Embodiment 99B. The method of any one of embodiments 85-98, wherein the fibrous tissue comprises a splanchnic nerve.

Embodiment 100. A method of making an implantable device, comprising:

attaching a feedthrough to a housing;

positioning a board assembly comprising a wireless communication system in the housing;

attaching the housing to a first nerve cuff layer;

attaching the first nerve cuff layer to a second nerve cuff layer comprising one or more electrodes; and

electrically connecting the one or more electrodes of the nerve cuff to the board assembly through the feedthrough.

Embodiment 101. The method of embodiment 100, wherein the feedthrough is attached to the housing prior to board assembly being positioned into the housing.

Embodiment 102. The method of embodiment 100, wherein the feedthrough is attached to the board assembly before the board assembly is positioned into the housing.

Embodiment 103. The method of any one of embodiments 100-102, comprising sealing the housing.

Embodiment 104. The method of any one of embodiments 100-103, comprising attaching an acoustic window to the housing.

Embodiment 105. The method of embodiment 104, wherein the acoustic window is attached to an open top of the housing after the board assembly is positioned within the housing.

Embodiment 106. The method of embodiment 104 or 105, comprising assembling the acoustic window by attaching a foil to a frame.

Embodiment 107. The method of any one of embodiments 100-106, comprising filling the housing with an acoustically conductive material.

Embodiment 108. The method of embodiment 107, wherein the acoustically conductive material is filled into the housing through a port on the housing, the method comprising sealing the port.

Embodiment 109. The method of any one of embodiments 100-108, comprising attaching the wireless communication system to the board assembly.

Embodiment 110. The method of any one of embodiments 100-109, wherein the wireless communication system comprises one or more ultrasonic transducers.

Embodiment 111. The method of any one of embodiments 100-110, wherein the board assembly comprises an integrated circuit electrically connected to the wireless communication system.

Embodiment 112. The method of any one of embodiments 100-111, wherein the housing is directly attached to the first nerve cuff layer.

Embodiment 113. The method of any one of embodiments 100-112, wherein an adhesive or a fastener is used to attach the housing to the first nerve cuff layer.

Embodiment 114. The method of any one of embodiments 100-113, wherein the first nerve cuff layer is helical.

Embodiment 115. The method of any one of embodiments 100-114, comprising attaching one or more handle portions to the body, the first nerve cuff layer, the second nerve cuff layer, or between the first nerve cuff layer and the second nerve cuff layer.

Embodiment 116. The method of any one of embodiments 100-115, comprising making the second nerve cuff layer by embedding one or more electrodes in a substrate material.

Embodiment 117. The method of any one of embodiments 100-116, comprising wrapping the second nerve cuff layer around a mandrel, and attaching the second nerve cuff layer to the first nerve cuff layer while the second nerve cuff layer is wrapped around the mandrel.

## Claims

1. A nerve cuff, comprising:

   a flexible helical substrate configured to at least partially wrap around a filamentous tissue comprising a nerve, and is configurable in (a) a flexed position by at least partially unwinding the helical nerve cuff, and (b) a relaxed position;

   one or more electrodes positioned along the length of the helical substrate; and

   a handle portion attached to the helical substrate configured to apply a force that configures the helical substrate in the flexed position.

2. The nerve cuff of claim 1, wherein the handle portion comprises a loop; and/or wherein the handle portion comprises a filament.

3. The nerve cuff of any one of the preceding claims, wherein the helical nerve cuff is configured to wrap around the nerve by at least one revolution; and/or wherein the helical nerve cuff is configured to wrap around the nerve by about 1.3 to about 1.7 revolutions.

4. The nerve cuff of any one of the preceding claims, wherein a portion of the handle portion is embedded within the helical substrate; and/or wherein the handle portion is attached to the nerve cuff at a position proximal to an end of the helical nerve cuff.

5. The nerve cuff of any one of the preceding claims, wherein the handle portion is attached to the nerve cuff at a position proximal to an end of the helical nerve cuff and the nerve cuff further comprises a second handle portion attached to the helical nerve cuff at a position proximal to a second end of the helical nerve cuff.

**6.** The nerve cuff of claim 5, wherein the first handle portion and the second handle portion are separated by a radial angle of about 90° to about 180° around a helical axis; and/or
wherein the first handle portion is attached to the second handle portion.

**7.** The implantable device of any one of the preceding claims, further comprising an additional handle portion attached to a middle position along the length of the helical nerve cuff.

**8.** The nerve cuff of any one of the preceding claims, wherein the helical substrate has a width that defines an inner surface, a first edge of the helical substrate, and a second edge of the helical substrate, and wherein at least a portion of the first edge contacts at least a portion of the second edge when the nerve cuff is in a relaxed position; or
wherein the helical substrate has a width that defines an inner surface, a first edge of the helical substrate, and a second edge of the helical substrate, and wherein the first edge does not contact the second edge when the nerve cuff is in a relaxed position.

**9.** The nerve cuff of any one of the preceding claims, wherein the at least one of the one or more electrodes is positioned along the length of the helical nerve cuff; and optionally,

wherein the at least one of the one or more electrodes positioned along the length of the helical nerve cuff is positioned on an inner surface of the helical nerve cuff; and/or
wherein the at least one of the one or more electrodes comprises one or more serpentine segments.

**10.** The nerve cuff of any one of the preceding claims, wherein the helical nerve cuff is configured to be unwound by at least one revolution.

**11.** The nerve cuff of any one of the preceding claims, wherein the helical nerve cuff comprises a right-handed helical portion; and/or

wherein the helical nerve cuff comprises a left-handed helical portion; and/or
wherein the helical nerve cuff comprises a right-handed helical portion joined to a left-handed helical portion.
the nerve cuff comprises a first helical portion joined to a second helical portion through a linear connecting member.

**12.** The nerve cuff of any one of the preceding claims, wherein the nerve cuff comprises one or more electrodes configured to emit an electrical pulse to the nerve; and/or

wherein the nerve cuff comprises one or more electrodes configured to detect the electrophysiological signal transmitted by the nerve; and/or
wherein the nerve cuff comprises two or more electrodes configured to detect the electrophysiological signal transmitted by the nerve.

**13.** The nerve cuff of any one of the preceding claims, wherein the nerve cuff comprises one or more electrodes configured to detect the electrophysiological signal transmitted by the nerve, and one or more electrodes configured to emit an electrical pulse to the nerve, wherein at least one of the one or more electrodes configured to emit an electrical pulse to the nerve is wider than at least one of the one or more electrodes configured to detect the electrophysiological signal transmitted by the nerve.

**14.** The nerve cuff of any one of the preceding claims, further comprising a mount attached to the helical substrate configured to receive a body and electrically connect the one or more electrodes positioned along the length of the helical substrate to the body; and, optionally,

wherein the body directly connects with the one or more electrodes; and, optionally,
wherein the mount comprises one or more feedthroughs configured to electrically connect the body to the one or more electrodes.

**15.** The nerve cuff of any one of the preceding claims, wherein the nerve is a splenic nerve.

FIG. 1A

FIG. 1B

FIG. 1C

FIG. 1D

FIG. 1E

FIG. 1F

FIG. 2A

FIG. 2B

FIG. 2C

FIG. 3A

FIG. 3B

FIG. 3C

FIG. 4A

FIG. 4B

FIG. 5A

FIG. 5B

FIG. 6A

FIG. 6B

FIG. 7

FIG. 8

FIG. 9

FIG. 10

FIG. 11A

FIG. 11B

FIG. 11C

FIG. 12

FIG. 13

At least partially unwind the helical nerve cuff
1402

Pass an end of the helical nerve cuff behind a fibrous tissue
1404

Wrap the helical nerve cuff around the fibrous tissue
1406

FIG. 14

Pass an end of the helical nerve cuff behind a fibrous tissue
1502

Pull the end of the helical nerve cuff, resulting in the helical nerve cuff becoming at least partially unwound
1504

Wrap the helical nerve cuff around the fibrous tissue
1506

FIG. 15

At least partially unwind the helical nerve cuff of an implantable device
1602

Pass an end of the helical nerve cuff opposite the device body behind a fibrous tissue
1604

Wrap the helical nerve cuff of the implantable device around the fibrous tissue
1606

FIG. 16

Pass an end of the helical nerve cuff opposite the device body behind a fibrous tissue
1702

Pull the end of the helical nerve cuff, resulting in the helical nerve cuff becoming at least partially unwound
1704

Wrap the helical nerve cuff of the implantable device around the fibrous tissue
1706

FIG. 17

EP 4 483 944 A2

Attach feedthrough to housing
1802

Position board assembly in housing
1804

Close open portion of housing with
acoustic window
1806

Fill housing with acoustically
conductive material
1808

Form first
substrate layer
1810

Attach housing on first substrate
layer
1812

Form second
substrate layer
1814

Attach housing on second substrate
layer to first substrate layer
1816

FIG. 18

1906

1904

1908

1910

1902

1912

**FIG. 19A**

1914b

1910

1906

1912

1918

1902

1908

1914a

1916

1920

**FIG 19B**

**FIG.19C**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62916709
- WO 2018009910 A2
- WO 2018009912 A2
- WO 2018009905 A2
- WO 2018009911 A2
- WO 2018009905 A
- WO 2018009908 A

- WO 2018009910 A
- WO 2018009911 A
- WO 2018009912 A
- US 2019028381 W
- US 2019028385 W
- WO 2019048647 A

**Non-patent literature cited in the description**

- **BERTRAND et al.** Beamforming Approaches for Untethered, Ultrasonic Neural Dust Motes for Cortical Recording: a Simulation Study. *IEEE EMBC,* August 2014